# EUROPEAN PATENT APPLICATION

(11) **EP 1 621 552 A1**
(43) Date of publication of application: **01.02.2006**
(21) Application number: 05013871.8
(22) Date of filing: 28.06.2005
(51) Int. Cl.: C07K 14/72, G01N 33/68, C12N 15/12, C12N 15/63

(54) **Human trace amine associated receptors (hTAAR)**

(30) Priority: 08.07.2004 EP 04103261
(71) Applicant: F.HOFFMANN-LA ROCHE AG, 4070 Basel (CH)
(72) Inventor: Ebeling, Martin, 79639 Grenzach-Wyhlen (DE); Hoener, Marius, 4056 Basel (CH); Lindemann, Lothar, 4057 Basel (CH)

(57) **Abstract**

The present invention provides a fingerprint sequence which is specific and selective for trace amine associated receptors (TAAR) forming a subfamily of G protein coupled receptors. The invention also provides the novel human polypeptides identified as members of this family, nucleic acids encoding said polypeptides, and vectors and host cells comprising the novel family members. In addition, the invention provides methods of identifying TAARs.

## Description

The present invention provides a fingerprint sequence which is specific and selective for trace amine associated receptors (TAAR) forming a subfamily of G protein coupled receptors. The invention also provides the human polypeptides identified as members of this family, nucleic acids encoding said polypeptides, and vectors, host cells and non-human animals comprising the novel family members. In addition, the invention provides methods of identifying human TAARs.

Trace amines (TA) are endogenous compounds structurally related to biogenic amines and are found in the mammalian nervous system in trace amounts. TAs are stored in nerve terminals and released together with classical biogenic amines. To date there is no evidence for the existence of synapses using TAs as their exclusive transmitter. Recently, receptors specifically binding trace amines were reported by Borowski et al (Trace amines: identification of a family of mammalian G protein-coupled receptors. Proc Natl Acad Sci USA (2001) 98(16):8966-71) and Bunzow et al (Amphetamine, 3,4-methylenedioxymethamphetamine, lysergic acid diethylamide, and metabolites of the catecholamine neurotransmitters are agonists of a rat trace amine receptor. Mol Pharmacol. 2001, 60(6):1181-8.). These receptors apparently represent a novel GPCR-family.

The dysregulation of trace amines were linked to various psychiatric disorders like depression (Sandler M. et al., Decreased cerebrospinal fluid concentration of free phenylacetic acid in depressive illness. Clin Chim Acta. 1979, 93(1):169-71; Davis BA and Boulton AA. The trace amines and their acidic metabolites in depression--an overview. Prog Neuropsychopharmacol Biol Psychiatry. 1994, 18(1):17-45.), schizophrenia (Potkin SG et al., Phenylethylamine in paranoid chronic schizophrenia. Science. 1979, 206(4417):470-1; Sandler M and Reynolds GP. Does phenylethylamine cause schizophrenia? Lancet. 1976, 1(7950):70-1.) and bipolar disorder (Boulton AA.: Some aspects of basic psychopharmacology: the trace amines. Prog Neuropsychopharmacol Biol Psychiatry. 1982;6(4-6):563-70; Sabelli HC et al., Clinical studies on the KM/29.04.2005 phenylethylamine hypothesis of affective disorder: urine and blood phenylacetic acid and phenylalanine dietary supplements. J Clin Psychiatry. 1986 Feb;47(2):66-70.). There has also been made a link between dysregulation of trace amines and attention-deficit hyperactivity disorder (Baker et al., Phenylethylaminergic mechanisms in attention-deficit disorder. Biol Phychiatry. 1991, 29(1):15-22), Parkinson's disease (Heller B and Fischer E., Diminution of phenethylamine in the urine of Parkinson patients. Arzneimittelforschung. 1973, 23(6):884-6.), migraine (D'Andrea G. et al., Elusive amines and primary headaches: historical background and prospectives. Neurol Sci. 2003, 24 Suppl 2:S65-7; D'Andrea, G. et al., Elevated levels of circulating trace amines in primary headaches. Neurology. 2004, 62(10):1701-1705.) and eating disorders (Wolf ME and Mosnaim AD. Phenylethylamine in neuropsychiatric disorders. Gen Pharmacol. 1983, 14(4):385-90; Branchek TA and Blackburn TP. Trace amine receptors as targets for novel therapeutics: legend myth and tact. Curr. Opin Pharmacol. 2003. 3(1):90-97.) such as i.e. obesity and anorexia as suggested by the very high structural similarity between PEA and amphetamin, which is considered the strongest anorexic compound known to date. (Samanin R, and Garattini S.: Neurochemical mechanism of action of anorectic drugs. Pharmacol Toxicol. 1993 Aug;73(2):63-8; Popplewell DA et al., A behavioural and pharmacological examination of phenylethylamine-induced anorexia and hyperactivitycomparisons with amphetamine. Pharmacol Biochem Behav. 1986 Oct;25(4):711-6.).

Therefore, there is a broad interest to increase the knowledge about trace amine receptors, especially to identify further trace amine receptors. However, examination of the literature and public database entries revealed inconsistencies in the naming of the TA receptors, e.g. the human receptor GPR102 (Lee et al., Discovery and mapping of ten novel G protein-coupled receptor genes. Gene. 2001, 275(1):83-91.) is also referred to as TA5 (Borowski et al Trace amines: identification of a family of mammalian G protein-coupled receptors. Proc Natl Acad Sci USA (2001) 98(16):8966-71)), and human 5-HT4Ψ (Liu et al., A serotonin-4 receptor-like pseudogene in humans. Brain Res Mol Brain Res. 1998, 53(1-2):98-103.) has also been named TA2Ψ (Borowski et al., Trace amines: identification of a family of mammalian G protein-coupled receptors. Proc Natl Acad Sci USA (2001) 98(16):8966-71). In addition, GPR57 (Lee et al., Cloning and characterization of additional members of the G protein-coupled receptor family. Biochim Biophys Acta. 2000, 1490(3):311-23.), GPR58 (Lee et al., Cloning and characterization of additional members of the G protein-coupled receptor family. Biochim Biophys Acta. 2000, 1490(3):311-23.) and PNR (Zeng et al., Cloning of a putative human neurotransmitter receptor expressed in skeletal muscle and brain. Biochem Biophys Res Commun. 1998, 242(3):575-8.) were so far not generally recognized as TA receptor. This inconsistencies cause confusion and uncertainty. Therefore, there is a strong need for a clear definition of this receptor family.

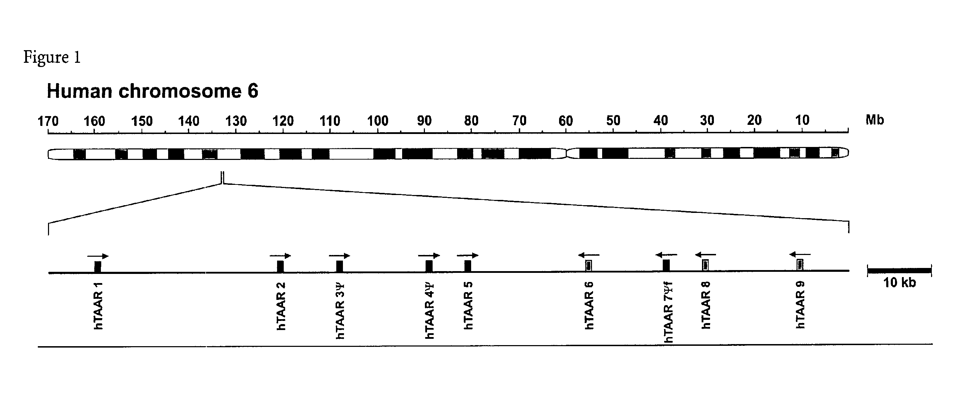

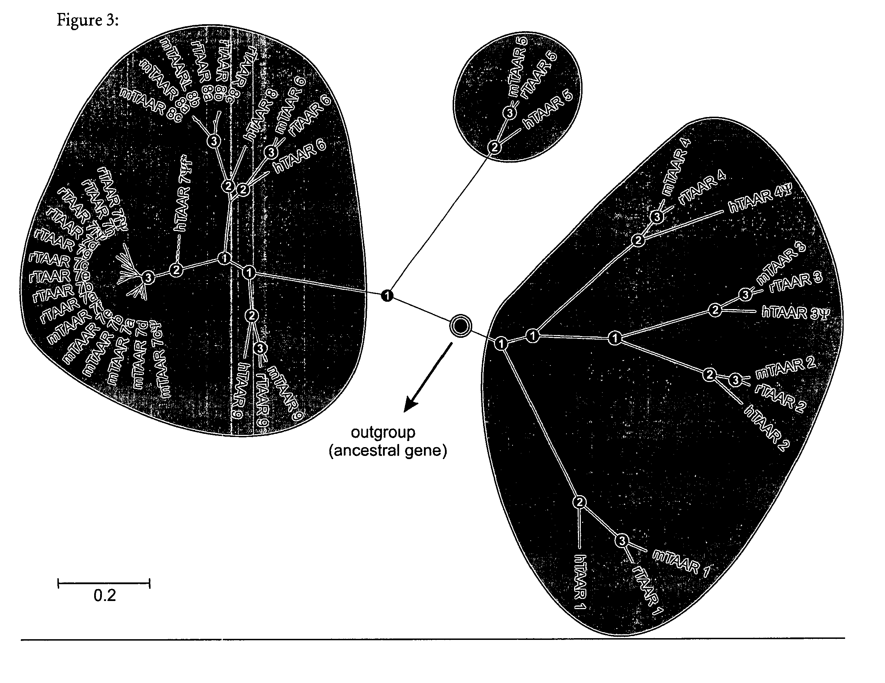

In order to resolve any ambiguity of the current naming a novel, uniform nomenclature is proposed with this invention which refers to these receptors as Trace Amine Associated Receptors (TAARs). This novel nomenclature reflects the finding that at least some TAARs do not respond to TAs at all, hence the term "associated". It covers all receptors of this GPCR family and is compatible with the different number of receptor genes in different species. The novel nomenclature is strictly based on the sequential order of the receptor genes on the respective human chromosome (see Fig. 1) as well as a detailed phylogenetic analysis (Fig. 3) of the receptor genes across different species. The nomenclature adheres to the following rules:
a) Any two (or three) genes that are orthologues, i.e. that were generated through a speciation event, should be labeled with the same number. Vice versa, two genes must not have the same number if they are not orthologous.
b) Genes that are paralogues, i.e. that were generated through a gene duplication event within the lineage of one species, should be distinguished by a letter suffix.
c) Examples: Genes hTAAR 5, rTAAR 5, mTAAR 5 are all orthologues.
   Genes mTAAR 8a,b,c in mouse are paralogues.
   Genes mTAAR 8a,b,c are all orthologues to hTAAR 8.

The present invention provides a novel polypeptide identified as TAAR family member, the use of said polypeptide as drug target, the polynucleotide sequence encoding said polypeptide, and vectors, host cells and non-human animals comprising said polynucleotide. Furthermore, the present invention pertains the fingerprint motif specific and selective for the TAAR family and the use of it.

The present invention provides a fingerprint motif comprising the sequence NSXXNPXXYXXXYXWF (SEQ. ID NO:1), wherein X is any natural occurring amino acid. The term "fingerprint", "fingerprint motif" or "fingerprint sequence" as used herein relates to an amino acid sequence which is specific and selective for the human GPCR subfamily TAAR. Preferably, the fingerprint motif is specific for functional TAARs. This TAAR fingerprint motif largely overlaps with the predicted transmembrane domain 7. The tryptophan residue in the context of the fingerprint motif is found exclusively in TAARs and not in any other known GPCR; rather, the corresponding sequence position is almost invariably occupied by polar or even charged amino acids in other GPCRs. The fingerprint motif may be used for identifying TAARs, preferably for identifying functional TAARs.

The present invention also provides a method of identifying human TAARs using the fingerprint sequence (SEQ. ID NO: 1). To be identified as a member of the human TAAR family a polypeptide may have 100% identity.

Furthermore, the present invention pertains a method of identifying TAARs of other species, preferably of mammalians, using the fingerprint sequence (SEQ. ID NO: 1). To be identified as a member of the TAAR family a polypeptide may have at least 75 % identity with the fingerprint sequence, preferably more than 87% identity, more preferably 100% identity. Preferably, the fingerprint motif varies the tyrosines, so is i.e. in rat the fingerprint sequence NSXXNPXX[Y,H]XXXYXWF and in mouse NSXXNPXX[Y,H]XXX[Y,F]XWF.

The fingerprint sequence may be used as a "query sequence" to perform a search against sequence databases to, for example to identify TAAR family members. Such searches can be performed using a pattern recognition program as for example fuzzpro of EMBOSS (Rice et al., EMBOSS: the European Molecular Biology Open Software Suite. Trends in Genetics, 2000, 16(6):276-277). A search for human TAAR may be performed with fuzzpro, NSXXNPXXYXXXYXWF as the required sequence, number of mismatches = 0, database = swissprot (release 43).

The human TAAR family contains nine members (hTAAR1 to hTAAR 9) wherein three of them are pseudogenes: hTAAR3Ψ, hTAAR 4Ψ and hTAAR7Ψ. All genes encoding the human TAAR family members are located in the same region of chromosome 6 (6q23). With the exception of hTAAR 2, which is encoded by two exons, the coding sequences of all TAAR genes are located in a single exon.

The term "gene" as used herein refers to any segment of DNA associated with a biological function. A gene is an ordered sequence of nucleotides located in a particular position on a particular chromosome that encodes a specific functional product.

The term "pseudogene" as used herein relates to an inactive gene which may have evolved by mutation events from an active ancestor gene. Inactive means that the gene is not translated to functional polypeptide.

The present invention provides an isolated or recombinant polypeptide comprising sequence SEQ. ID NO: 5. Said polypeptide was identified as trace amine associated receptor (TAAR) and was named hTAAR2.

The term "polypeptide sequence" (e.g., a protein, polypeptide, peptide, etc.) as used herein relates to a polymer of amino acids comprising naturally occurring amino acids.

The present invention further pertains the polynucleotide sequence comprising SEQ. ID NO: 4 that encodes the polypeptide hTAAR2.

The term "polynucleotide sequence" (e.g., a nucleic acid, polynucleotide, oligonucleotide, etc.) as used herein relates to a polymer of nucleotides comprising nucleotides A,C,T,U,G.

The term "isolated" means altered "by the hand of man" form the natural state. If an "isolated" composition or substance occures in nature, it has been changed or removed from its orignial environment, or both. For example, a polynucleotide or a polypeptide naturally present in a living animal is not "isolated", but the same polynucleotide or polypeptide separated form the coexisting materials of its natural state is "isolated" as the term is employed herein.

The term "recombinant" when used with reference, e.g. to a polynucleotide or polypeptide typically indicates that the polynucleotide or polypeptide has been modified by the introduction of a heterologuous (or foreign) nucleic acid of the alteration of a native nucleic acid, or that the protein or polypeptide has been mdified by the introduction of a hteerologous amino acid.

The present invention also provides a polynucleotide comprising sequence SEQ. ID NO 16. Said polynucleotide was identified as a gene fragment of a trace amine associated receptor and was named as hTAAR7Ψf.

The other members of the TAARs family (hTAAR1, hTAAR3, hTAAR4, hTAAR5, hTAAR6, hTAAR8 and hTAAR9) were previously described (Borowski et al Trace amines: identification of a family of mammalian G protein-coupled receptors. Proc Natl Acad Sci USA (2001) 98(16):8966-71; Bunzow et al. 2001; WO 00/60081), however the published sequences have minor errors (see Table 1). The present invention also provides the correct polynucleotide sequences of hTAAR1 (SEQ. ID NO: 2), hTAAR3Ψ (SEQ. ID NO: 6), hTAAR4Ψ (SEQ. ID NO: 9), hTAAR5 (SEQ. ID NO: 12). This invention pertains further the correct amino acid sequences of hTAAR1 (SEQ. ID NO: 3), and hTAAR5 (SEQ. ID NO: 13).

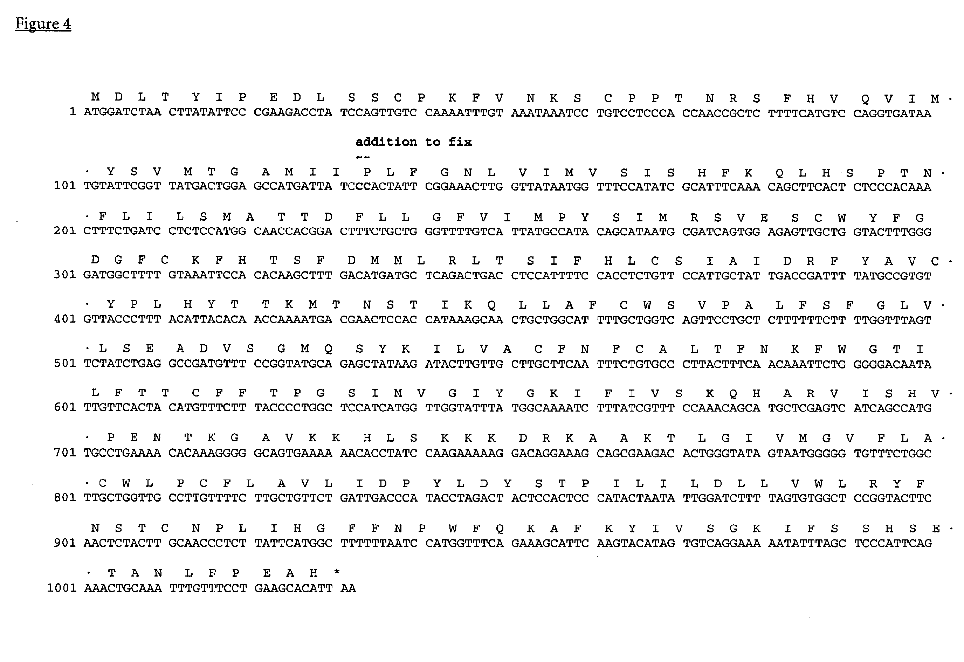

The present invention also provides the repaired nucleotide sequences of hTAAR3Ψ (SEQ. ID NO: 7) and hTAAR4Ψ (SEQ. ID NO: 10). This invention further provides the polynucleotide sequences encoded by the fixed nucleotide sequence hTAAR3 (SEQ. ID NO: 8) and hTAAR4 (SEQ. ID NO: 11). The term "repaired gene" or "fixed gene" as used herein relates to a pseudogene whose sequence was altered in a way that the gene becomes active. The polynucleotide sequence of i.e. hTAAR3 is inactive due to a mutation that caused an early stop codon. The sequence was repaired by insertion of two nucleotides at position 133-134 (see Figure 4).

The present invention also provides isolated or recombinant polypeptides comprising an amino acid sequence which comprise the fingerprint sequence and differs from the sequences SEQ. ID NOs: 3, 13, 15, 18, 20.

In a further aspect the invention relates to the use of the polypeptides of the invention as drug target. Preferably, the polypeptides of the invention are used as a drug target for identifying compounds useful in depression therapy, in schizophrenia therapy, in migraine therapy, or in therapy of attention-deficit hyperactivity disorder or eating disorder as anorexia or obesity. The drug target may be suitable for the design, screening and development of pharmaceutically active compounds.

One embodiment of the invention relates to the polypeptide having a polypeptide sequence SEQ. ID NO: 5 as drug target. Preferably, the polypeptides of the invention are used as a drug target for identifying compounds useful in depression therapy, in schizophrenia therapy, in migraine therapy, or in therapy of attention-deficit hyperactivity disorder or of eating disorder as anorexia or obesity.

Another embodiment of the invention relates to the receptors having an amino acid sequence which comprise the fingerprint sequence and differs from the sequences SEQ. ID NOs: 3, 13, 15, 18, 20 as drug target. Preferably, these receptors are drug target for identifying compounds useful in depression therapy, in schizophrenia therapy, in migraine therapy, or in therapy of attention-deficit hyperactivity disorder or of eating disorder as anorexia or obesity.

The term "polypeptide of the invention" relates to the novel polypetides provided in the present invention, i.e. hTAAR2.

The present invention also pertain vectors comprising polynucleotides of the invention, host cells which are transduced with said vectors and the production of the polypeptides of the invention by recombinant techniques. Preferably, the vector comprises the polynucleotide comprising SEQ. ID NO: 4 and preferably, the host cells are transduced with said vector.

Host cells can be genetically engineered (i.e., transduced, transformed or transfected) to incorporate expression systems or portion thereof for polynucleotides of the present invention. Introduction of the vector into host cells can be effected by methods described in many standard laboratory manuals, such as Davis et al., Basic methods in molecular biology (1986) Davis JM (ed.): Basic cell culture, sec. edition. Oxford University Press 2002, ISBN: 0199638535; R. Ian Freshney: Culture of Animal Cells: A Manual of Basic Technique, fourth edition. John Wiley & Sons (Sd) 1999, ISBN: 0471332852 and Sambrook et al., Molecular cloning: a laboratory manual, 2. Ed., cold Spring Harbour Laboratory Press, Cold Spring Harbor, N.Y (1989) such as calcium phosphate transfection, DEAE-dextran mediated transfection, transvectin, microinjection, cationic lopid-mediated transfection, electrporation, transduction or infection.

A host cell may be mammalian cell as HEK 293, CHO, COS, HeLa, neuronal, neuroendicrinal, neuroblastomal or glial cell lines like SH-SY5Y, PC12, HN-10 (Lee HJ, Hammond DN, Large TH, Roback JD, Sim JA, Brown DA, Otten UH, Wainer BH.: Neuronal properties and trophic activities of immortalized hippocampal cells from embryonic and young adult mice. J Neurosci. 1990 Jun;10(6):1779-87.), IMR-32, NB41A3, Neuro-2a, TE 671, primary neuronal or glia cells from mammals like rat or mouse, Xenopus oocytes, bacterial cells such as streptococci, staphylocooci, E. *coli, Streptomyces* and *Bacillus subtilis* cells; fungal cells such as *Saccharomyces cerevisiae* and *Aspergillus* cell; insect cells such as *Drosophila* S2 and *Spodoptera* Sf9 cells and plant cells.

A great variety of expression systems can be used. Such a system include, among others, chromosomal, episomal and virus -derived systems, i.e. vectors derived from bacterial plasmids, from bacteriophage, from transposons, from yeast episoms, from yeast chromosomal elements, from viruses such as *baculovirus,* papova viruses, such as SV40, vaccinia viruses, adenovirus, fowl pox virus, pseudorabies, retroviruses and vectors derived from combinations thereof, such as those derived from plasmids and bacteriophage genetic elements, such as cosmids and phagemids. The expression systems may contain control regions that regulate as well as engender expression. Generally, any system or vector suitable to maintain, progagate or express polynucleotides to produce a polypeptide in a host may be used. The appropriate nucleotide sequence may be inserted into an expression system by any of a variety of well-known and routine techniques, such as, for example, those set forth on Sambrook et al., Molecular cloning: a laboratory manual, 2. Ed., Cold Spring Harbour Laboratory Press, Cold Spring Harbour, N.Y. (1989) or Bowosky et al. 2001 supra.

The present invention further provides a transgenic non-human animal comprising polynucleotide encoding a polypeptide of the invention. Preferably, the transgenic non-human animal comprises a polynucleotide comprising SEQ. ID NO:4.

The transgenic non-human animal may be any non-human animal known in the art. Preferably, the transgenic non-human animal is a mammal, more preferably the transgenic non-human animal is a rodent. Most preferably, the transgenic animal of the invention is a mouse.

Methods of producing a transgenic non-human animal are well known in the art such as for example those set forth on Hogan, B.C., F; Lacy, E, *Manipulating the Mouse Embryo: A Laboratory Manual*. 1986, New York: Cold Spring Harbor Laboratory Press; Hogan, B., et al., Manipulating the mouse embryo. 1994, Second Edition, Cold Spring Harbor Press, Cold Spring Harbor and Joyner, A. (ed.): Gene Targeting - A Practical Approach Second Edition. Practical Approach Series, Oxford University Press 1999.

The polypeptides of the invention may be employed in a screening process for compounds which bind the receptor and which activate (agonists) or inhibit (antagonists) activation of the receptor polypeptide of the present invention or compounds which modulate receptor function e.g. by acting on receptor trafficking.

The present invention provides a method for identifying compounds which bind to the polypeptide of the invention comprising:
a) contacting the polypeptide of the invention with a candidate compound and
b) determing whether compound binds to the polypeptide of the invention.

The invention also provides a method for identifying compounds which have a stimulatory or inhibitory effect on the biological activity of polypeptide of the invention or its expression comprising
a) contacting the polypeptide of the invention with a candidate compound and
b) determining if said compound has modulated the function or activity of the polypeptide of the invention.

In general, such screening procedures as described above involve producing appropriate cells which express the receptor polypeptide of the present invention on the surface thereof, however, the receptor expressed in appropiate cells may also be localized intracellularly. Such cells include but are not limited to cells from mammals, Xenopus, yeast, Drosophila or E. coli. Culture conditions for each of these cell types is specific and is known to those familiar with the art. Cells expressing the receptor (or cell membrane containing the expressed receptor) are then contacted with a test compound to measure binding, or stimulation or inhibition of a functional response.

One screening technique includes the use of cells which express receptor of this invention (for example, transfected CHO cells or HEK293) in a system which measures extracellular pH or intracellular calcium changes caused by receptor activation. In this technique, compounds may be contacted with cells expressing the receptor polypeptide of the present invention. A second messenger response, e.g., signal transduction, pH changes, PI hydrolyse, GTP-γ-[³⁵S] release or changes in calcium level, is then measured to determine whether the potential compound activates or inhibits the receptor.

Another method involves screening for receptor inhibitors by determining inhibition or stimulation of receptor-mediated cAMP and/or adenylate cyclase accumulation. Such a method involves transfecting a eukaryotic cell with the receptor of this invention to express the receptor on the cell surface. The cell is then exposed to potential antagonists in the presence of the receptor of this invention. The amount of cAMP accumulation is then measured. If the potential antagonist binds the receptor, and thus inhibits receptor binding, the levels of receptor-mediated cAMP, or adenylate cyclase, activity will be reduced or increased.

Another methods for detecting agonists or antagonists for the receptor of the present invention is the yeast based technology as described in U.S. Patent 5,482,835.

The assays may simply test binding of a candidate compound wherein adherence to the cells bearing the receptor is detected by means of a label directly or indirectly associated with the candidate compound or in an assay involving competition with a labeled competitor. Further, these assays may test whether the candidate compound results in a signal generated by activation of the receptor, using detection systems appropriate to the cells bearing the receptor at their surfaces. Inhibitors of activation are generally assayed in the presence of a known agonist and the effect on activation by the agonist by the presence of the candidate compound is observed. Standard methods for conducting such screening assays are well understood in the art.

Examples of potential polypeptide of the invention antagonists include antibodies or, in some cases, oligonucleotides or proteins which are closely related to the ligand of the polypeptide of the invention, e.g., a fragment of the ligand, or small molecular weight molecules such as e.g. metabolites of neurotransmitters or of amino acids, which bind to the receptor but do not elicit a response, so that the activity of the receptor is prevented.

Having now generally described this invention, the same will become better understood by reference to the specific examples, which are included herein for purpose of illustration only and are not intended to be limiting unless otherwise specified, in connection with the following figures.

### Figures

Figure 1 shows the chromosomal localization of TAARs genes in human. The relative position of TAAR genes on the chromosomes is indicated by boxes, representing the single coding exons of the respective genes. For hTAAR 2, which is encoded by two exons, the box indicates the position of the second coding exon harboring more than 95% of the open reading frame. The width of the boxes is not to scale with regard to the length of the respective coding sequences, and the colors indicate a distinction in receptor subgroups as discussed in the text. Arrows on top of the boxes indicate the orientation of the TAAR genes (→: ORF in direction of the forward strand, ←: ORF in direction of the reverse strand). (f = fragment;Ψ= Pseudogene)

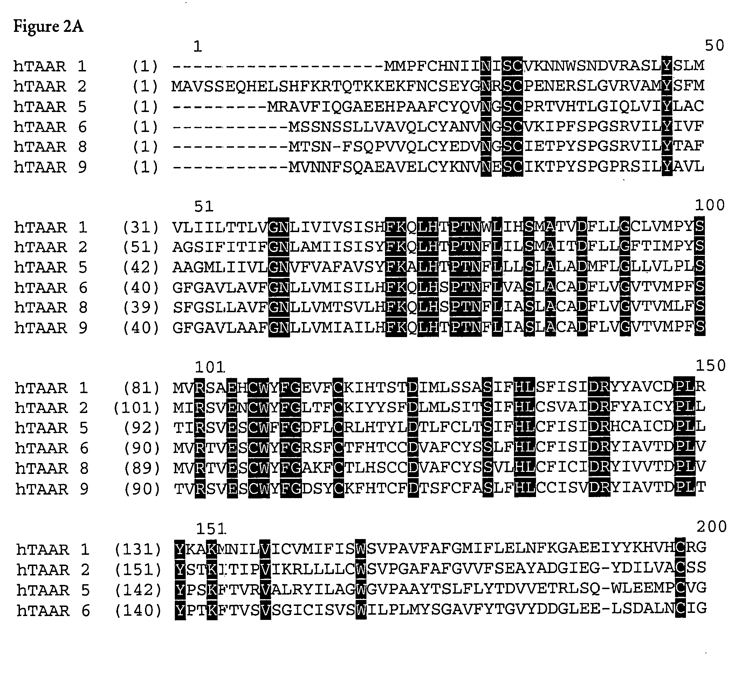

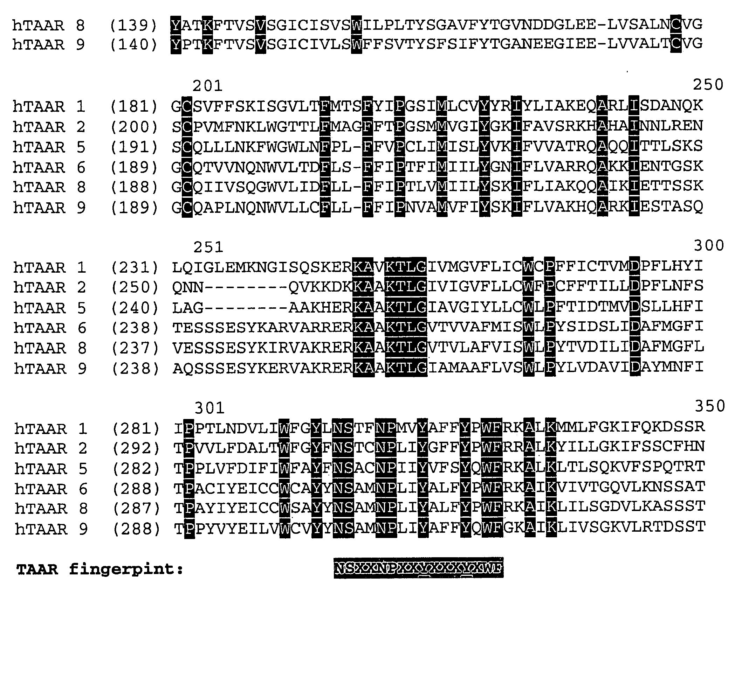

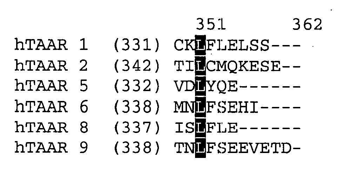

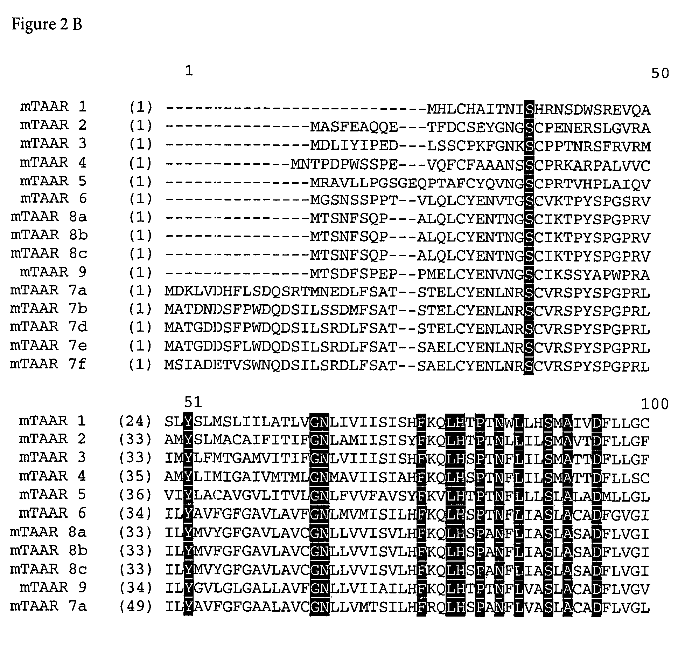

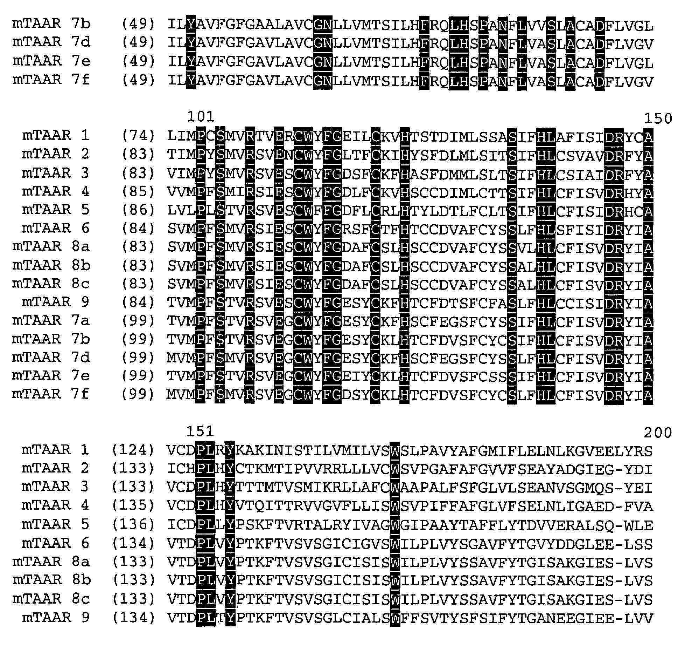

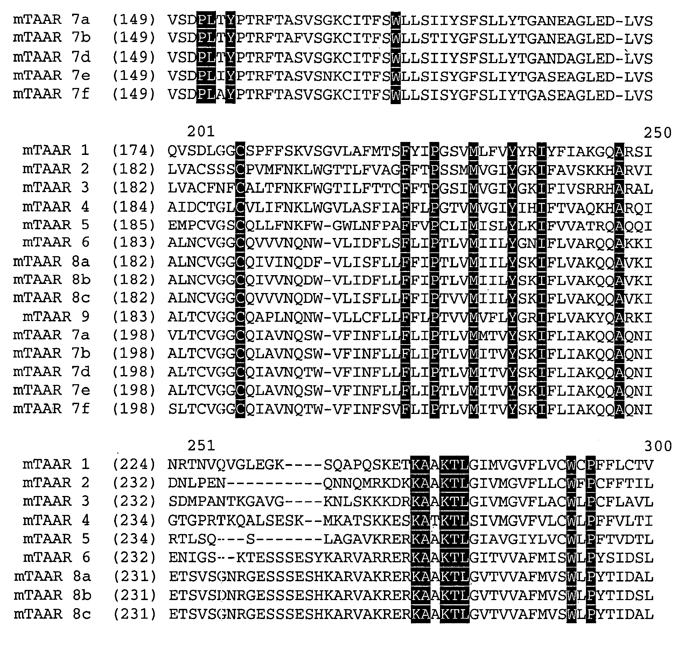

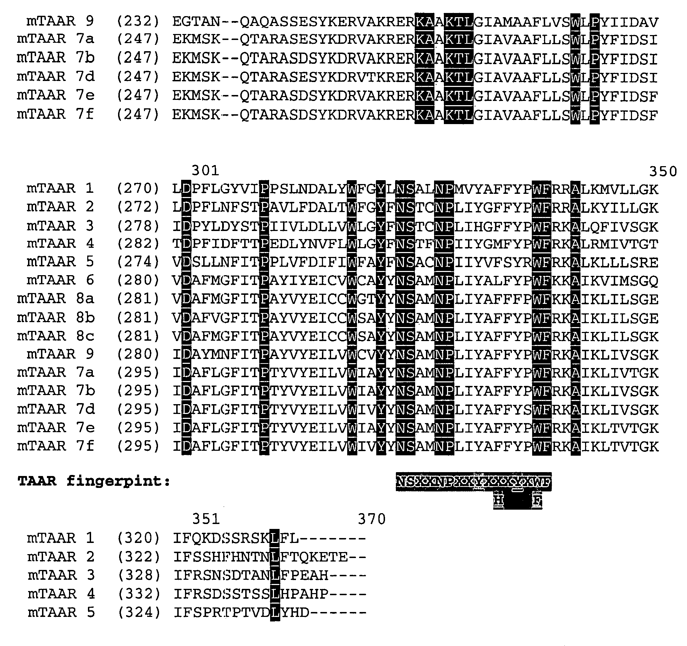

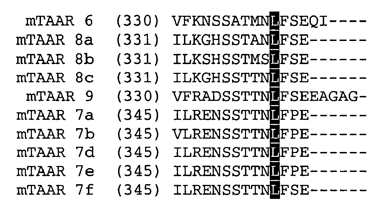

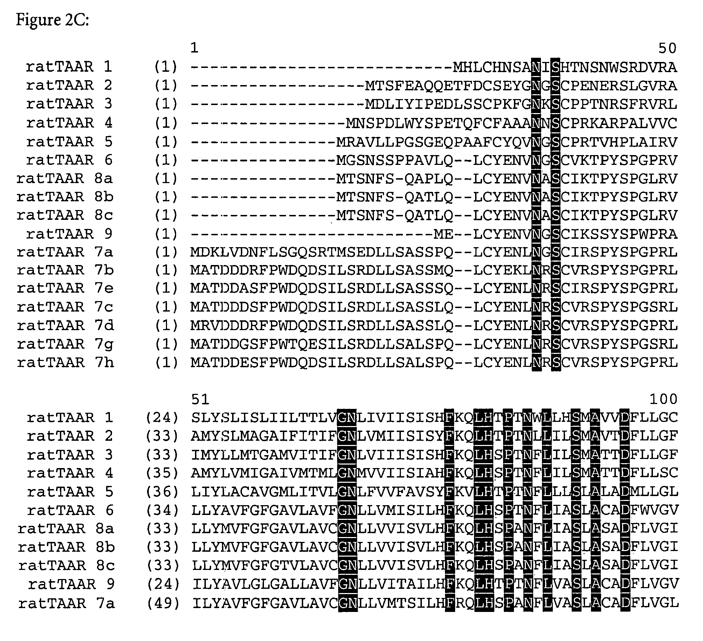

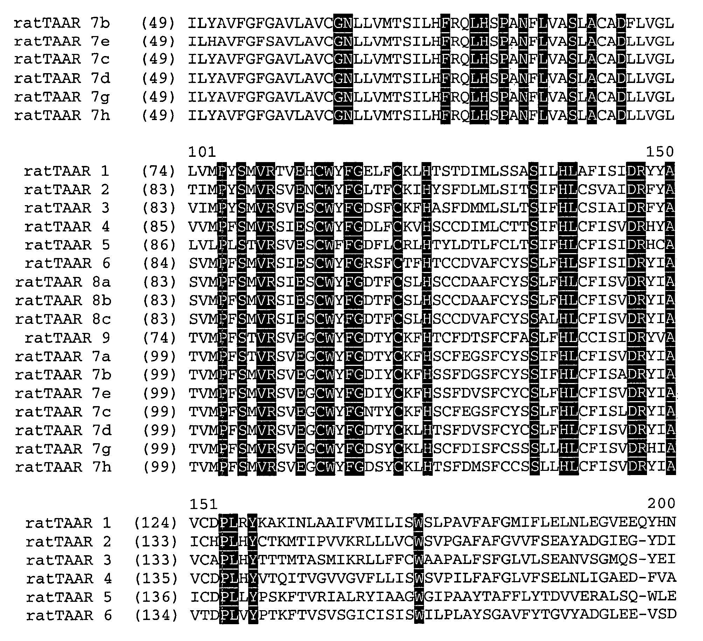

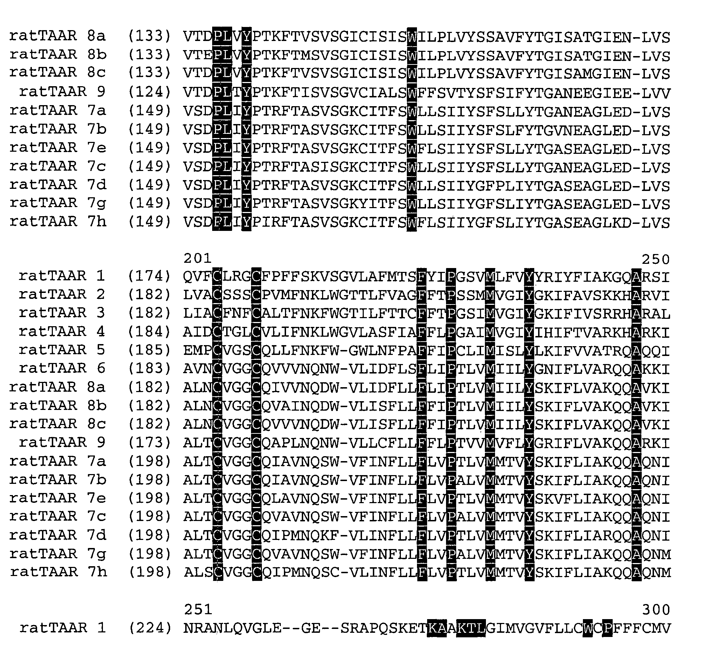

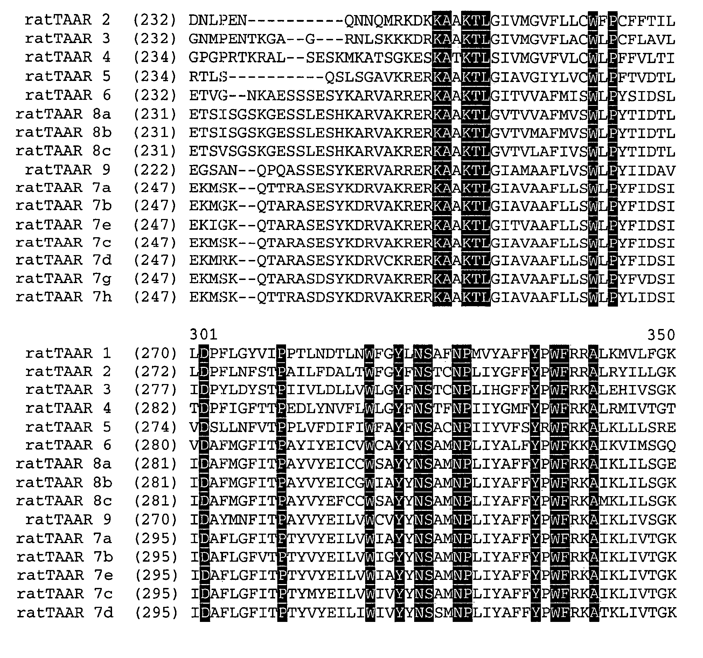

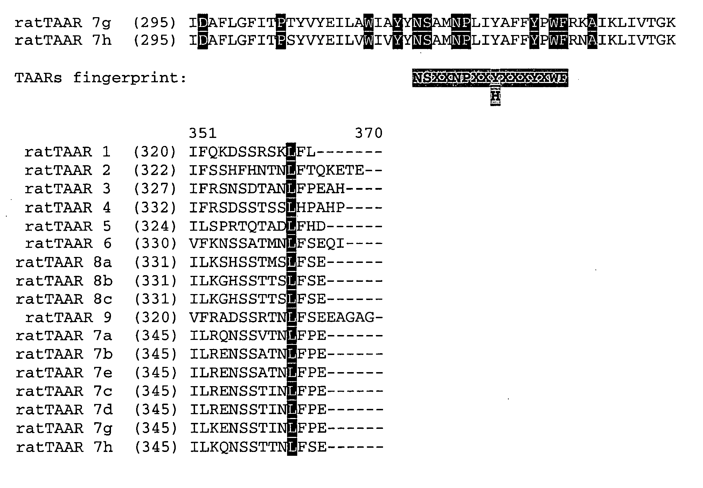

Figure 2 shows an alignment of all functional TAARs. Amino acid residues conserved in all human, rat and mouse TAARs are highlighted by black shading. The characteristic TAAR fingerprint motif is located in TM VII.
   A: alignment of functional human TAARs. **Predicted TM Positions: hTAAR1** (TM I: 26-46, TM II: 60-80, TM III: 99-109, TM IV: 137-157, TM V: 189-209, TM VI: 253-273, TM VII: 288-308); **hTAAR 2** (TM I: 49-71, TM II: 80-102, TM III: 129-151, TM IV: 163-185, TM V: 208-230, TM VI: 263-285, TM VII: 300-322); **hTAAR 5** (TM I: 36-58, TM II: 71-93, TM III: 108-130, TM IV: 150-172, TM V: 203-225, TM VI: 253-270, TM VII: 285-307); **hTAAR 6** (TM 1: 33-53, TM II: 69-89, TM III: 108-128, TM IV: 148-168, TM V: 203-223, TM VI: 260-276, TM VII: 283-302); **hTAAR 8** (TM I: 37-59, TM II: 66-88, TM III: 103-125, TM IV: 145-167, TM V: 198-220, TM VI: 258-280, TM VII: 290-312); **hTAAR 9** (TM I: 38-58, TM II: 69-89, TM III: 107-128, TM IV: 148-168, TM V: 198-218, TM VI: 260-280, TM VII: 295-315)
   B: alignment of functional mouse TAARs.
   C:alignment of functional rat TAARs.
Figure 3 shows the phylogenetic relationship of TAAR genes in human, rat and mouse based on their DNA sequence.
   Subsequent gene duplication events are indicated by numbers at the branching points ( 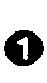 : Gene duplication from a putative common ancestor; 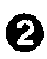 : Speciation leading to separate primates and rodent lineages; 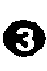 : Gene duplication leading to mouse and rat species). The outgroup was placed based on sequence comparison to the human 5-HT₄ receptor. The summary of TAAR genes into distinct subfamilies is indicated by boxes with grey shading. Ψ: Pseudogene; *: Gene fragment with closest similarity to rat TAAR 7h in the human genome resembling remnants of rodent TAAR 7 paralogues. Scale: JTT protein distances.
Figure 4 shows the "repair" of the pseudogene hTAAR 3 (old name GPR57ψ): The two bps CC in position 133-134 of the fixed ORF were added, which repairs the otherwise early stop codon TCA.

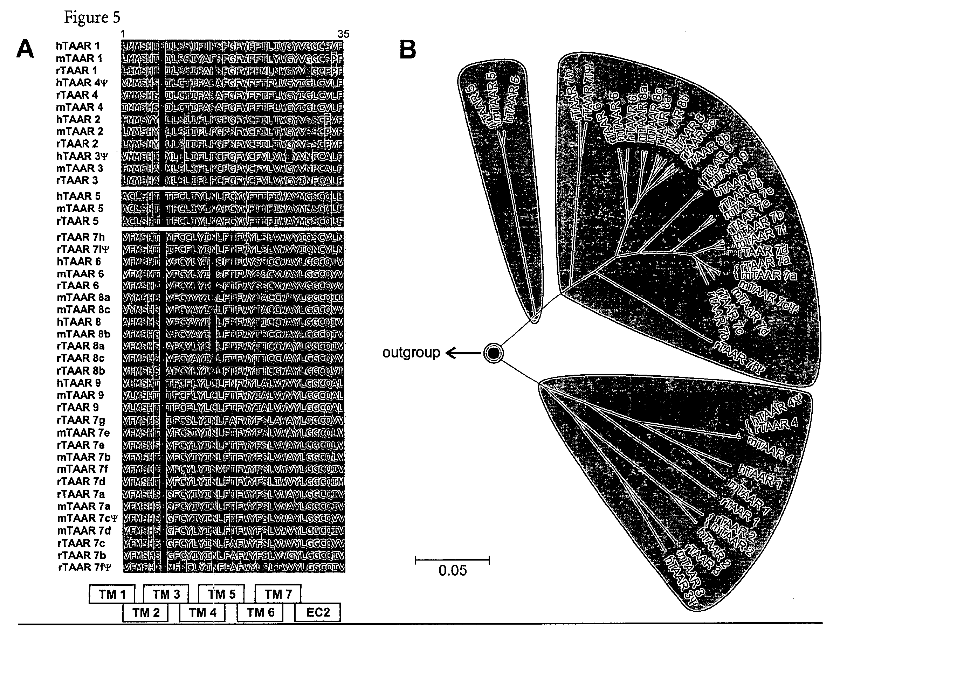

Figure 5 shows the relationship of TAARs based on the properties of the ligand pocket vectors (amino acid whose residues probably take part in the ligand binding, determined by calculation model).
   A: The Ligand Pocket Vectors (LPVs) of human, rat and mouse TAAR proteins, as predicted according to Kratochwil et al. (2004). Individual TAARs are ordered such that proteins with closest similar ligand binding pockets are placed next to each other. The physicochemical properties of the amino acid residues are indicated by colored shading (blue: hydrophobic/aromatic residues; green: aliphatic polar residues; red/plum: positively/negatively charged residues; orange/yellow: glycine/proline residues, potentially inducing kinks in the TM domains; pink: histidine; light blue: tyrosine). The position of the respective amino acids in the transmembrane domains (TM 1-7) as well as the extracellular loop II (EC II) is indicated by boxes in the lower part of the figure. For the precise position of predicted TM regions please refer to legend of figure 2.
   B: Hierarchical tree representation of the pharmacophore similarity of the ligand binding pockets of TAAR proteins. The following proteins were included into the analysis as "repaired pseudogenes", modification in parenthesis: hTAAR 3Ψ (insertions: C135-, A136-), hTAAR 4Ψ (pointmutation: T411A, deletions: -605G, -750A), mTAAR 7cΨ (insertion: C513-), rTAAR 7fΨ (pointmutation: G515A), rTAAR 7iΨ (insertion: A90-). Scale bar: Pharmacophore diversity units.

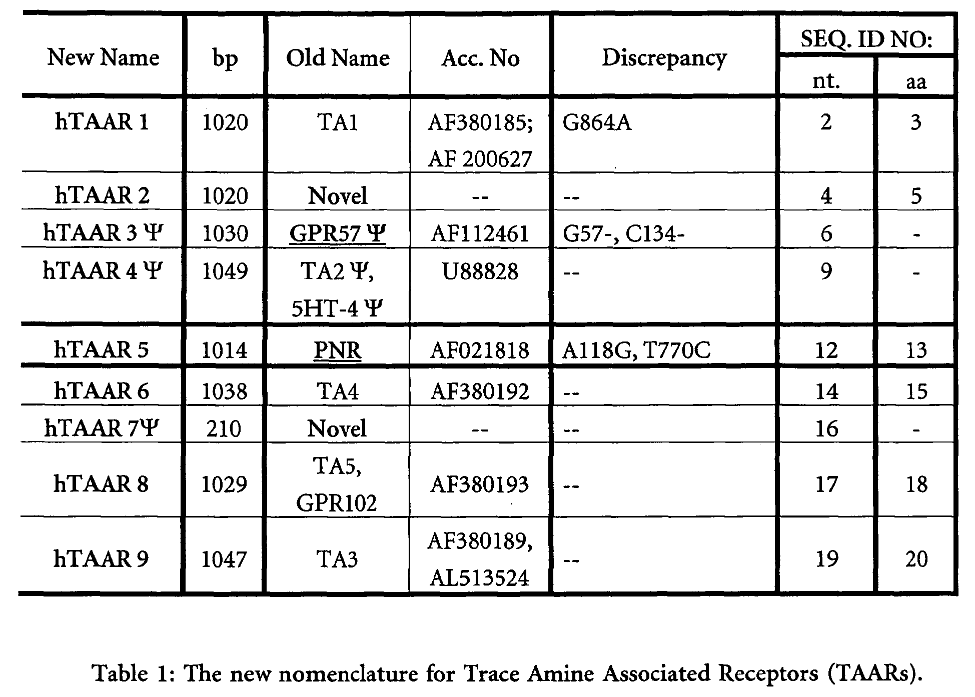

All genes for which discrepancies between published sequence data and the sequences provided by this invention were detected, are underligned. Genbank accession numbers refers to published genes and the discrepancies between the published sequences and the sequences provided by the present invention. Cloning and sequence analysis of TAAR genes was carried out according to standard protocols employing genomic or cDNA from human (derived from total human brain total RNA, Stratagene). (nt=nucleotide sequence; aa= amino acid sequence)

### Examples

Commercially available reagents referred to in the examples were used according to manufacturer's instructions unless otherwise indicated.

### Example 1: Identification of TAAR family members

The TAAR genes were identified by comparison of the previously published TAAR receptor genes with the human, rat and mouse genomic sequence information available from Genbank (...see also Table 1) employing standard algorithms like BLAST.

Based on this sequence information the TAAR genes were amplified by means of PCR from either genomic DNA or cDNA of the respective species. Were not further specified, all procedures were carried out basically described in: Sambrook, J., Fritsch, E.F., und Maniatis, T. (1989). Molecular Cloning: A laboratory manual (New York: Cold Spring Harbor Laboratory Press). All reagents were of highest available purity, and all solutions and reagents were sterilized prior to use unless otherwise stated.

For this purpose oligonucleotide primers (table 2) were designed based on the TAAR coding sequences which had been derived from the genomic sequence information available at Genbank. The primers were designed such that the amplicons contain the entire open reading frames. The primer were designed using the VectorNTI software (Vector NTI version 9.0.0, Informax) following standard rules for PCR primers, mainly: a) Primers should have a length of 18-25 nt b) a G/C content of about 50% c) should not contain inverted repeats longer than 3 nt d) should carry an G or C at least at the 5' end e) should have no simple repetitions of the same nts more than 3, and f) the annealing temperatures (TM) of primers used in the same PCR reaction should differ as little as possible (for details see for example: McPherson M.J., Hames B.D., Taylor G.R. (eds.): PCR 2 - A Practical Approach. The practical approach series, Oxford University Press, 1995, ISBN: 0-19-963424-6). The annealing temperatures of the primer oligonucleotides were calculated based on rules described in: Breslauer KJ, Frank R, Blocker H, Marky LA.: Predicting DNA duplex stability from the base sequence. Proc Natl Acad Sci U S A. 1986 Jun;83(11):3746-50 (These rules assume that the sequences are not symmetric and contain at least one G or C and have a length of minimally 8 nts). Oligonucleotides were ordered from Microsynth (Microsynth AG, Schützenstrasse 15, 9436 Balgach, Switzerland) in HPLC purified quality.

**Table 2: Primer for cloning of hTAARs.**

| Receptor | Primer names | Sequence (5' → 3') | TM | SEQ. ID NO | Comment |
|---|---|---|---|---|---|
| Human | | | | | |
| hTAAR 1 | hTAAR 1_5_01 | atgatgcccttttgccac | 59.2 | 21 | |
| | hTAAR 1_3_01 | ttatgaactcaattccaaaaataatttac | 58.4 | 22 | |
| hTAAR 2 | humGPR58_5_0 6 | catctacatctggggtatcttg | 55.9 | 23 | Cloned from cDNA |
| | humGPR58_3_0 6 | tgcttcaatttattcatgcag | 56.3 | 24 | |
| hTAAR 3 (GPR57) | humGPR57_5_0 1 | atggatctaacttatattcccgaa | 55.9 | 25 | Cloned from cDNA |
| | humGPR57_3_0 1 | ttaatgtgcttcaggaaacaaa | 56.3 | 26 | |
| hTAAR4 | hTAAR 4_5_01 | atgatgaatttgcctgacc | 55.5 | 27 | |
| | hTAAR 4_3_01 | ctaagcatgggcagaaaac | 55.6 | 28 | |
| hTAAR 5 | humPNR_5_01 | atgagagctgtcttcatcca | 55.4 | 29 | |
| | humPNR_3_01 | tcattcttggtacaaatcaacag | 56.6 | 30 | |
| hTAAR6 | hTAAR6_5_01 | atagcagcaattcatccc | 58.3 | 31 | |
| | hTAAR 6_3_01 | ttatatatgttcagaaaacaaattcatg | 56.9 | 32 | |
| hTAAR8 | hTAAR8_5_01 | ataccagcaatttttccc | 57.1 | 33 | |
| | hTAAR 8_3_01 | ttattctaaaaataaactaatggttgatg | 56.8 | 34 | |
| hTAAR9 | hTAAR 9_5_01 | atggtgaataatttctcccaag | 56.9 | 35 | |
| | hTAAR 9_3_01 | ttaatctgtctctacttcttcagaaaat | 56.7 | 36 | |

hTAAR2: The main reason of cloning the hTAAR 2 from cDNA was to assure whether the transcript of the corresponding genes would in fact contain the two exons. Independent from these experiments, the two coding exons have been cloned separately from genomic DNA. (Tm: melting temperature in °C calculated according to Breslauer KJ, Frank R, Blocker H, Marky LA.: Predicting DNA duplex stability from the base sequence. Proc Natl Acad Sci U S A. 1986 Jun;83(11):3746-50.; 5 in the primer name indicates a 5' primer and 3 in the primer name indicates a 3' primer).

The actual PCR reactions had a total volume of 50 µl and the following composition: Template equaling 5-100 ng genomic DNA or cDNA equivalent to 100-200 ng of total RNA (sources specified below), 200 nM oligonucleotide primer, 1.5 mM MgCl₂ (Invitrogen), 200 mM dNTPs each (Invitrogen), 1x concentrated PCR reaction buffer (Invitrogen) and 5 U/reaction recombinant Taq DNA polymerase (Invitrogen). The PCR reactions were assembled under a sterile working bench with UV irradiated equipment, and a) preparation of template material, b) assembly of PCR reactions, c) running the PCR reactions and agarose gel electrophoresis, and d) plasmid preparation were each performed in separated rooms in order to avoid any possible cross contamination of reagents with PCR amplified material or plasmid DNA. The PCR reactions were assembled in 200 µl PCR cups (Eppendorf) including Taq DNA polymerase at room temperature (RT) and transferred to the thermocycler (Geneamp 9700 with gold block, Applied Biosystems) preheated to 95°C. The temperature profile was adjusted as follows:

| | |
|---|---|
| 95°C | 2 min |
| (95 °C: 30 sec, annealing temperature: 30 sec, 72°C: extension time) x cycle number: | |
| 72°C | 5 min |
| 4°C | max. 6 hrs |

| | |
|---|---|
| **annealing temperature**: the Tm (melting temperature) of the oligonucleotide primer with the lower Tm value for the PCR reaction (calculated as specified above) -1°C was used as annealing temperature. Example: 2 primers with Tm primer 1 = 55°C, Tm primer 2 = 57°C ⇒ annealing temperature = 54°C. | |
| **extension time** was calculated by length of amplicon (in kb) x 1 min. | |
| **cycle number**: For each gene several PCR reactions were run in parallel with cycle numbers between 25 - 40. All PCR reactions were subsequently analyzed by agarose gel electrophoresis, and the PCR reaction with the lowest cycle number still producing a clearly visible PCR product of the correct size was used for subsequent cloning. | |

The PCR products were analyzed by agarose gel electrophoresis using 1% ultraPure agarose (GibcoBRL) made up in TAE buffer (Invitrogen). Agarose gels were run in PerfectBlue Horizontal Mini Electrophoresis Systems (Pequlab Biotechnologie GmbH, Erlangen, Germany) according to standard protocols (Sambrook et al., 1989). Agarose gels were stained with ethidium bromide, PCR products were visualized on a UV transilluminator (Syngene, Cambridge, UK), and the size of the PCR products was analyzed in comparison with the molecular weight standard 1 kb DNA ladder (Invitrogen). PCR products of the expected sizes were cut out from the gels with sterile scalpels (Bayha, Tuttlingen, Germany) and extracted from the agarose gel slices using the QIAquick Gel Extraction Kit (QIAGEN AG, Basel, Switzerland) following the instructions of the manufacturer.

The extracted PCR products were precipitated with a cold ethanol/sodium acetate precipitation (Sambrook et al., 1989), and DNA pellets were dissolved in a volume of 10 µl of 10 mM Tris/HCl pH 7.5. The PCR products in this solutions were subsequently cloned using the TOPO cloning kit for sequencing (Invitrogen; kits containing the vectors pCR4-TOPO and pCR2.1-TOPO were used) following the instructions of the manufacturer. Ligations were transformed into TOP10 chemically competent bacteria (included in the TOPO cloning kit for sequencing) following the instructions of the manufacturer and subsequently plated on LB agar plates containing 100 µg/ml ampicillin (Sigma, Division of FLUKA Chemie GmbH, Buchs, Switzerland) and incubated at 37°C over night. The bacterial colonies were analyzed by a method called "miniprep PCR": Colonies were picked with a sterile inoculation loop (Copan Diagnostic S.P.A., Brescia, Italy) and transferred to new LB agar plates containing 100 µg/ml ampicillin. The same inoculation loops were subsequently transferred individually into 50 µl 10 mM Tris/HCl pH 7.5 in 1.5 ml Eppendorf Cups (Eppendorf) in order to transfer the bacteria which had remained on the loop into the solution. This bacterial suspensions were subsequently heated to 95°C for 5 min, and 1 µl pf the resulting bacterial lysates per 50 µl PCR reaction was used as template for PCR reactions with primers flanking the multiple cloning site of the plasmids pCR4-TOPO and pCR2.1-TOPO (T7 and M13 reverse, sequences: Primer "T7": 5' - cgggatatcactcagcataat - 3', primer "M13 reverse": 5' - caggaaacagctatgacc - 3'). PCR reactions were assembled as described above and run with the following temperature profile:

| | |
|---|---|
| 95°C | 2 min |
| (95 °C: 30 sec, 50°C: 30 sec, 72°C: 1 min) x 30 cycles | |
| 72°C | 5 min |
| 4°C | max. 6 hrs. |

The PCR products were analyzed by agarose gel electrophoresis as described above. Bacterial colonies for which cloned DNA fragments of the expected sizes could be detected by the method outlined above were used for subsequent plasmid preparation.

For plasmid preparations bacterial colonies identified to carry inserts of the expected sizes by "miniprep PCR" were used to inoculate bacterial liquid cultures in LB medium containing 100 µg/ml ampicillin, and cultures were incubated on a horizontal shaker at 37°C over night (Sambrook et al., 1989). Plasmids were isolated from the bacterial liquid cultures using the HiSpeed Plasmid Maxi Kit (QIAGEN AG, Basel, Switzerland) following the instructions of the manufacturer. Plasmid concentrations were determined by measuring the OD at 260 nm using an UV/VIS spectrophotometer ultrospec 3300 pro (Amersham Biosciences Europe GmbH, Otelfingen, Switzerland). The size of inserts in the isolated plasmids was analyzed by means of restriction digests using an restriction endonuclease i.e. EcoRI (New England Biolabs, products distributed in Switzerland by Bioconcept, Allschwil, Switzerland); EcoRI restriction sites are flanking the multiple cloning sites in both vectors pCR4-TOPO and pCR2.1-TOPO, therefore the cloned inserts can be released from the plasmids by EcoRI restriction digests. Of each plasmid 0.5 µg were digested with EcoRI in a total volume of 20 µl following the recommendations of the manufacturer. Restriction digests were analyzed by agarose gel electrophoresis as described above. Plasmids which turned out to carry inserts of the expected sizes as revealed by restriction analysis were subjected to DNA sequence analysis, carried out by an external company (Microsynth AG, Schützenstrasse 15, 9436 Balgach, Switzerland).

The DNA sequences of the cloned TAAR gene open reading frames, as revealed by DNA sequence analysis, were compared to either previously published sequences and/or to the sequence of the TAAR genes retrieved from the genome sequence information. Potential errors in the DNA sequence information which could have been introduced by the PCR reaction were eliminated by comparing the sequences of the two to three DNA plasmids which were cloned each from independent PCR reactions per TAAR gene: Since the probability of the same PCR error to occur at the very same position in independent PCR reactions is basically zero, the alignment of the independent sequences for each gene revealed the very correct DNA sequence.

Template material: We paid particular attention to use standardized template material derived, were applicable, from the same inbred strains from which the genome sequence information available at Genbank was derived. Mice of the inbred strain C57BL/6, or genomic DNA derived from this mouse strain, was purchased from Jackson Laboratory (600 Main Street, Bar Harbor, Maine 04609 USA), rats of the inbred strain Crl:Wi was purchased from Charles River Laboratories France (Les Oncins, France; belongs to Charles River Laboratories, Inc.,251 Ballardvale Street, Wilmington, MA 01887-1000,USA). Genomic DNA of tail biopsies taken from adult Crl:Wi rats was prepared basically according to: Laird PW, Zijderveld A, Linders K, Rudnicki MA, Jaenisch R, Berns A.: *Simplified mammalian DNA isolation procedure.* Nucleic Acids Res. 1991 Aug 11;19( 15):4293. The DNA concentration was determined by measuring the OD at 260 nm as described above and the DNA concentration was adjusted to 100 ng/µl. The DNA was stored in aliquots at 4°C. Human genomic DNA was purchased from Stratagene.

cDNAs were either commercially purchased (human: Human brain, cerebellum Marathon-Ready cDNA, BD Biosciences Clontech), or made from total RNA. RNA was either commercially purchased (human: human adult total brain, male, total RNA, Stratagene, Stratagene Europe, P.O. Box 12085 1100 AB Amsterdam, The Netherlands) or extracted from postnatal day 3 C57BL/6 mouse or Crl:Wi rat pups as described below.

### RNA isolation:

RNA was isolated from tissue samples basically according to: Chomczynski, P. und Sacchi, N.: Single-step method of RNA isolation by acid guanidinium thiocyanate-phenol-chloroform extraction. (Chomzynski P, Sacchi N., Analytical Biochemistry (1987) Single-Step Method of RNA Isolation by Acid Guanidinium Thiocyanate-PhenolChloroform Extraction 162, 156-159. Basic rules and principles for working with RNA are described in detail in Sambrook et al. (1989).

Rat or mice pups were killed by decapitation. The total brains were removed, weighed, and transferred to a 5 ml glass douncer and homogenized in the 10x volume of Trizol Reagent (Invitrogen). Total RNA was extracted from this homogenate following the instructions of the manufacturer. The RNA pellet derived from each half brain was dissolved on 200 µl 10 mM Tris/HCl pH 7.0. Traces of genomic DNA were removed by DNAseI digest (addition of 10 µl 100 µl MgCl₂ and 5 µl RNAse free DNAse I, 10³ U/µl, Roche; incubation at 37°C for 1 hr). DNAse I was removed from the solution by phenol/chloroform extraction (H₂O saturated phenol pH 7.0, Sigma) according to Sambrook et al. (1989). The RNA was precipitated by cold ethanol/sodium acetate precipitate (Sambrook et al., 1989), dissolved in 10 mM Tris/HCl pH 7.0, and the RNA concentration was determined by measuring the OD at 260 nm as described above. This RNA preparation was used for the synthesis of cDNA.

cDNA synthesis: In an 1.5 ml Eppendorf cup was mixed: 2 µg total RNA in a total volume of 11µl, 1 µl 0.5 µg/µl oligo dT12-18 (Invitrogen). The mixture was heated to 70°C for 10 min, placed on ice, and the following reagents were added:

4 µl 5x reaction buffer (supplied with enzyme), 2 µl DTT (supplied with enzyme), 1 µl dNTPs (10 mM each, Arnersham), 0.5 µl RNAse inhibitor RNAse-OUT (Invitrogen), 0.8 µl Superscript II Reverse Transcriptase (Invitrogen). The reaction was briefly vortexed, incubated for 1 hr at 42°C, the enzyme was inactivated by incubating at 70°C for 10 min, and the reaction was adjusted to a final volume of 100 µl with 10 mM Tris/HCl pH 7.0. The cDNA was stored in aliquots at -80°C prior to use.

In general, particular measures were taken to rule out sequence errors by the PCR reaction and by polymorphisms introduced by the template material: a) Cycle number: For each TAAR gene several PCR reactions with different cycle numbers were run, and the PCR reactions with lowest possible cycle number which still delivered a specific product in amounts sufficient for cloning were used. b) Several independent clones per gene: Each TAAR genes was cloned 2 or 3 times from independent PCR reactions, and the correct sequence was deduced from an alignment of the sequences of these independently generated plasmids: Since the likelihood of a PCR error to occur at the very same position in several independent PCR reactions is basically zero, the alignment of the sequence of these plasmids generates the very correct sequence of the gene. c) Standardized template material: Since differences between inbred strains can introduce significant inconsistencies in sequence information, we also paid particular attention in the choice of the template material employed for the cloning of TAAR genes: Mouse genomic DNA was purchased from Jackson Laboratory (600 Main Street, Bar Harbor, Maine 04609 USA) genomic DNA of the inbred strain C57BL/6 was used, since this strain was employed for the mouse genomic sequence database at Genbank), human genomic DNA was purchased from Stratagene, and rat genomic DNA was isolated from a rat of the inbred strain Crl:Wi purchased from Charles River Laboratories France (Les Oncins, France; belongs to Charles River Laboratories, Inc.,251 Ballardvale Street,Wilmington, MA 01887-1000,USA; the rat strain Crl:Wi was selected, since this strain was employed for the rat genomic sequence database at Genbank). For the synthesis of cDNA rat and mouse whole brain total RNA was isolated from a postnatal day 3 pup of either a Crl:Wi rat or a C57BL/6 mouse, employing the Trizol reagent following the recommendations of the manufacturer. Human total brain RNA was purchased from Stratagene. The cDNA was synthesized using SuperScript II Reverse Transcriptase from Invitrogen and dNTPs from Amersham following the recommendations of the manufacturers.

### Example 2: Cell culture & generating stable cell lines

a) Subcloning: For expression of TAAR receptors in mammalian cell lines, DNA fragments carrying the entire TAAR open reading frames were subcloned from the pCR4-TOPO or pCR2.1-TOPO vectors (Invitrogen) to pIRES-NEO2 (Rees S et al., Bicistronic vector for the creation of stable mammalian cell lines that predisposes all antibioticresistant cells to express recombinant protein. Biotechniques. 1996 Jan;20(1):102-4, 106, 108-10).
   To this end, DNA fragments carrying the entire TAAR open reading frames were removed from the respective TOPO vectors by restriction digest with i.e. EcoRI and subsequent purification of the ~1kb fragments by agarose gel electrophoresis and gel extraction as described above. In parallel, the pIRES-NEO2 vector was linearized with EcoRI and dephosphorylated with shrimp alkaline phosphatase (Roche) following the instructions of the manufacturer. The DNA fragments carrying the entire TAAR open reading frames were ligated into the linearized pIRES-NEO2 vector using T4 DNA ligase (New England Biolabs) by mixing 30 ng of linearized pIRES-NEO2 vector, 100-300 ng of DNA fragments carrying the respective TAAR coding sequence, 1x ligation buffer (final concentration; provided with the enzyme as 10x concentrate) and 1 µl of T4 DNA ligase in a total volume of 20 µl. The reaction was proceeded at room temperature for 2-3 hrs, and the ligation was transformed into chemically competent TOP10 cells as described for the TOPO ligations. Plating of transformed bacteria and picking of bacterial clones was performed as described earlier for the PCR cloning of TAAR genes from genomic and cDNA. The picked bacterial clones were used to inoculate 5 ml liquid cultures in LB containing 100 µg/ml ampicillin. These liquid cultures were used for mini plasmid preparations using the QIAprep Miniprep Kit (QIAGEN), following the instructions of the manufacturer. The purified pIRES-NEO2 derived plasmid constructs were analyzed for the presence of inserts of the expected sizes by restriction analysis using EcoRI and agarose gel electrophoresis. Plasmids which were identified to carry inserts of the expected sizes were subjected to DNA sequence analysis carried out by Microsynth in order to make sure for the correct orientation and sequence of the inserts. Plasmids carrying the correct inserts in the correct orientation were used for expression of the respective TAARs in mammalian cell lines.
b) Cell culture: Basic cell culture handling and techniques used are described in Davis JM (ed.): Basic cell culture, sec. edition. Oxford University Press 2002, ISBN: 0199638535. TAARs were expressed in HEK cells (ATCC number: CRL-1573; described in Graham, F.L., Smiley, J., Russell, W.C., und Nairn, R. (1977). Characteristics of a human cell line transformed by DNA from human adenovirus type 5. Journal of General Virology 36, 59-74). The culture medium consisted of DMEM with Glutamax I, with sodium pyruvate, with pyridoxine, with 4500 mg/l glucose, Invitrogen Cat. # 31966-021; Penicillin/Streptomycin; fetal calf serum heat inactivated 10%. Cells were passaged at a ratio of 1:10-1:30 using Trypsin/EDTA (Invitrogen Cat. # 25300-062).
c) Stable cell lines: For the generation of stably transfected cell lines HEK cells were transfected with the pIRES-NEO2 expression vectors of the respective TAARs by using Lipofectamin 2000 transfection reagent and Optimem 1 reduced serum medium with Glutamax (Invitrogen Cat. # 51985-026) following the recommendations given in the Lipofectamine 2000 datasheet. After 24 hr post transfection cells were trypsinated and transferred into 90 mm tissue culture dishes (Nunc) at dilutions between 1:10 - 1:300 in culture medium supplemented with 1 mg/ml G418 which was renewed on a daily basis. After 7-10 days pot transfections well isolated clones were observed which were picked when they had reached a diameter of about 3 mm. Picking of clones was performed by detaching the entire clone from the culture dish using a 1 ml Gilson pipette, trypsinizing the clone in a 1.5 ml Eppendorf cup using Trypsin/EDTA (Invitrogen Cat. # 25300-062) and plating them into a 48 well dish, from where the cells were subsequently expanded by transferring them to increasing culture surface areas (48 well -12 well - 60 mm etc.). Whenever cells were transferred to a new plate they were carefully trypsinized. As soon as single clones were selected, the concentration of G418 in the culture medium was reduced to 500 µg/ml.

After sufficient expansion of the individual clones they were tested in a functional assay with the Amersham cAMP Biotrak Enzymeimmunoassay (EIA) System, using either the trace amines (p-tyramine, β-phenylethylamine (β-PEA), tryptamine, octopamine) or a selection of compounds (5-HT Serotonin, dopamine, norepinephrine and histamine). The success rate for generating stably transfected cell lines was about 1 out of 3 clones tested. This high success rate was made possible by the use of the pIRES vector, from which the TAARs are expressed in bicistronic transcripts carrying the TAAR CDSs and the NEO^{R} CDS in the same mRNA molecule.

| | Human TAAR 1 EC₅₀µ*M*) | Max(%) |
|---|---|---|
| β-PEA | 0.30 +/- 0.09 | 100 |
| p-TYR | 1.07 +/- 0.10 | 100 |
| OCT | 10.29 +/- 1.55 | 100 |
| TRY | 46.87 +/- 4.43 | 100 |
| Dopamine | 15.78 +/- 7.66 | 50 |
| Serotonin, 5-HT | > 50.000 | |
| Norepinephrine | > 50.000 | |
| Histamine | > 50.000 | |
| N-methyl-β-PEA | 0.16 +/- 0.04 | 100 |
| N-methyl-p-tyramine | 2.05 +/- 1.06 | 100 |

**Table 3:** Pharmacology of hTAAR1. The amount of cAMP produced by HEK293 cells stably expressing the indicated receptor in response to stimulation by each of the listed compounds was measured using the cAMP Biotrak EIA System (Amersham). Shown are the EC₅₀ values in µM (means of at least three independent experiments; EC₅₀ values are calculated from experiments carried out with 12 concentrations each in duplicates evenly distributed in the range of 30 µM - 0.1 nM). The maximal response is represented as percentage of the maximal cAMP levels induced by p-TYR. The human TAAR 1 used in this study was a chimera constructed by adding an N-terminal influenza hemaglutinin viral leader sequence and replacing selected regions with the corresponding rat TAAR 1 sequences (i.e. N- and C-terminus and 3^{rd} intracellular loop). This chimera was obtained by David K. Grandy, University Portland (J.R. Bunzow, M.S. Sonders, S. Arttamangkul, K.S. Suchland, S.G. Amara, T.S. Scanlan, D.K. Grandy. PHARMACOLOGY AND PHYSIOLOGY OF TRACE AMINE RECEPTORS Program No. *515.4. 2002 Abstract Viewer*/*Itinerary Planner.* Washington, DC: Society for Neuroscience, 2002. Online.). In the experiments with Dopamine EC₅₀ values were calculated based on a maximal cAMP level of 50% compared to the level reached by p-Tyramine.

For all other hTAARs than hTAAR 1 a minimum of 10 independent clones were tested. If no responder to either trace amines or selected top compounds could be detected it was concluded that the respective TAAR was not responsive to the compounds at all under the defined test conditions.

**cAMP assay:** cAMP was assayed using the cAMP Biotrak Enzymeimmunoassay (EIA) System from Amersham as described in the product manual, following the socalled Non-acetylation EIA procdure. The general procedure is also described i.e. in Notley-McRobb L et al. (The relationship between external glucose concentration and cAMP levels inside Escherichia coli: implications for models of phosphotransferase-mediated regulation of adenylate cyclase. Microbiology. 1997 Jun;143 ( Pt 6):1909-18) and Alfonso A, de la Rosa L et al. (Yessotoxin, a novel phycotoxin, activates phosphodiesterase activity. Effect of yessotoxin on cAMP levels in human lymphocytes. Biochem Pharmacol. 2003 Jan 15;65(2):193-208.)

In the protocol the following minor modifications were introduced: After stimulation of HEK cells in 96 well plates the reaction was stopped by applying equal volume of pre cooled (-20°C) ethanol. Lysis of cells was not achieved by applying the lysis buffer supplied with the kit, but with transferring the 96 well plates to -80°C for a minimum of 6 hrs (up to several days). Thereafter the contents of the 96 well plates was dried at 50°C over night, and the material remaining in the 96 well plates was resuspended in 200 µl of the assay buffer (the buffer used for resuspending all reagents of the kit). The resuspension were assayed in serial dilutions in assay buffer.

### Phylogenetic Analysis

The human, mouse and rat genome sequences (human: NCBI reference draft 34, July 2003; mouse: NCBI draft 32, October 2003; rat: Rat Genome Sequencing Consortium draft 3, June 2003) were screened, employing an in-house genomic sequence analysis resource for the identification of all TAAR genes and in particular for the presence of previously unknown potential members of the TAAR family. This screen led to the identification of 44 TAAR genes overall in human, mouse and rat, among them 22 novel genes (including 2 pseudogenes and a human TAAR 7 gene fragment) and 11 genes with discrepancies to previously published sequences. The comparison of simple DNA and protein sequence, as well as of more complex parameters like the GPCR pharmacophore relationship of the ligand pocket vectors and the phylogenetic relationship of the TAAR genes with the genome sequences strongly support the conclusion that the TAAR receptor listed in Figure 3 represents the complete TAAR receptor family in human, mouse and rat. The identification of TAAR genes based on the genome sequence information was largely facilitated by the fact that all TAARs except TAAR 2 are encoded by single exons..

In this context the arrangements of the TAAR genes in the human, mouse and rat genomes were analyzed (for human see Fig. 1). The genes mapped to very compact regions spanning a total of about 109 kb in human (chromosome 6q23.1), 192 kb in mouse (chromosome 10A4) and 216 kb in rat (chromosome 1p12; all chromosomal regions as assigned by LocusLink), with no other annotated or predicted genes in the same chromosomal regions. With the exception of TAAR 2, which is encoded by two exons, the coding sequences of all TAAR genes are located in a single exon and are very similar in their length of about 1 kb throughout all three species (range: 999-1089 bp). It is interesting to note that the region 6q23.1 on human chromosome no. 6 is known as schizophrenia susceptibility locus (Levinson et al.,: *Multicenter linkage study of schizophrenia candidate regions on chromosomes 5q, 6q, 10p, and 13q: schizophrenia linkage collaborative group III.* Am J Hum Genet. 2000 Sep;67(3):652-63. Epub 2000 Aug 02.; Martinez et al., *Follow-up study on a susceptibility locus for schizophrenia on chromosome* 6q. Am J Med Genet. 1999 Aug 20;88(4):337-43.) which underlines the potential of TAARs as drug targets for psychiatric disorders.

In human TAAR 1-5, as well as TAAR 6, 8 and 9 were found to be arranged in two blocks of genes with an inverse orientation of the open reading frames. The most striking difference between human and rodents is the complete absence of any functional TAAR 7 paralogues from the human genome, which represent almost half of the entire TAAR family in rat and for which we detected only a degenerate gene fragment in human with closest similarity to rTAAR 7h. In addition, TAAR 3 and 4 are functional receptors in rodents, but pseudogenes in human. The pseudogenization of hTAAR 3Ψ and 4Ψ likely is a recent event, since these genes are overall very well preserved with only two and three bp changes in the coding sequences, respectively, rendering them non-functional. The different total number of nine TAAR 7 paralogues in rat (including two pseudogenes, rTAAR 7fΨ and rTAAR 7iΨ) and six TAAR 7 paralogues in mouse (including one pseudogene, mTAAR 7cΨ) represents a remarkable inter-rodent difference within a family of such closely related genes, which is reflected in strong predicted ligand binding differences between individual mouse and rat TAARs determined by the GPCR pharmacophore relationship of the ligand pocket vector analysis (see below). The strong differences detected in the predicted ligand binding pockets of receptors with highly similar overall protein sequence indicates that the overall amino acid sequence identity is no suitable measure for predicting the degree of similarity of mouse and rat TAARs with regard to their pharmacological profile, a fact requiring close attention for the development of behavioral animal models.

Triggered by the compact chromosomal arrangement ofTAAR genes in human and rodents and the high number of very similar genes within each species, the phylogenetic relationship of TAAR genes was analyzed across all three species. The phylogenetic analysis shows that the TAAR genes originate in a putative common ancestor from which they evolved through a total of eight gene duplication events, leading to a set of nine genes before the primate and rodent lineage split (Fig. 2; gene duplication events from the common ancestor, speciations leading to human and primate lineage, and gene duplications within the rodent lineage are each collectively indicated by numbers 1, 2 and 3, respectively). In seven cases, these genes were further duplicated into orthologeous sets of three by the two speciation events leading to human, mouse and rat. The rodent TAAR 7 and 8 orthologues evolved by further, independent duplications within the rat and mouse lineages. It is important to note that these events can only have occurred in the sequential order outlined above.

The overall structure of the phylogenetic tree immediately suggested to us the distinction of the TAAR family into three subgroups (indicated by grey boxes in Fig. 3). As discussed in detail below it turned out that the concept of subgroups is not an arbitrary distinction inferred just by the overall DNA sequences, but it reflects significant functional differences.

There are a total number of five pseudogenes in human, rat and mouse. All pseudogenes could be transformed into functional proteins by introduction of minor changes. Following a description of the most obvious and likely mutational events that might have caused the pseudogenizations: hTAAR 3Ψ became a pseudogene most likely due to a deletion of two bps (CA) at position 135 causing an early termination. A pointmutation (A411T) turning a Cys into a stop codon and two insertions (G605, A750) might have caused the pseudogenisation of hTAAR 4Ψ. rTAAR 7fΨ has two obvious differences to all the other TAAR 7 members of 12 additional bps at position 726, probably caused by an insertion event and of a pointmutation (A515G) turning a Trp into a stop codon. The second rat pseudogene rTAAR 7iΨ misses 10 bps at position 90 causing an early termination, and mTAAR 7cΨ misses 15 bps at position 895 (intracellular loop 3) and 7 bps at position 513 causing an early termination. In summary, within the TAAR family pseudogenisation seems to be caused by all possible mutational events like pointmutations, deletion and insertions. The events seem to have occurred fairly recently due to the highly conserved overall sequence. The pharmacology of such repaired pseudogenes might give insights into the importance of TAARs in the context of adaptation processes during evolution.

## Claims

1. An isolated or recombinant polypeptide comprising SEQ ID NO: 5.

2. Use of the polypeptide according claim 1 as drug target.

3. Use of the polypeptide according claim 1 as drug target in schizophrenia therapy.

4. Use of the polypeptide according claim 1 as drug target in migraine therapy.

5. Use of the polypeptide according claim 1 as drug target in depression therapy.

6. Use of the polypeptide according claim 1 as drug target therapy of eating disorders.

7. Use of the polypeptide according claim 1 as drug target for identifying compounds useful in therapy of attention deficit hyperactivity disorder.

8. An isolated or recombinant polynucleotide comprising SEQ. ID NO: 4.

9. A vector comprising the polynucleotide according to claim 8

10. A host cell comprising the vector according to claim 9.

11. A transgenic non-human animal comprising the polynucleotide according to claim 8.

12. A fingerprint motif comprising the sequence NSXXNPXXYXXXYXWF wherein X is any natural occurring amino acid.

13. Use of a sequence comprising the fingerprint motif according claim 12 for identifying TAARs.

14. An isolated or recombinant polypeptide comprising a sequence which comprises the fingerprint motif of claim 12 and differs from the sequences SEQ. ID NOs: 3, 13, 15, 18, 20.

15. A polypeptide according to claim 14 as drug target.

16. A polypeptide according to claim 14 as drug target for identifying compounds useful in schizophrenia therapy.

17. A polypeptide according to claim 14 as drug target for identifying compounds useful in migraine therapy.

18. A polypeptide according to claim 14 as drug target for identifying compounds useful in depression therapy.

19. A polypeptide according to claim 14 as drug target for identifying compounds useful in therapy of eating disorder.

20. A polypeptide according to claim 14 as drug target for identifying compounds useful in therapy of attention deficit hyperactivity disorder.

21. A method for identifying compounds which bind to a polypeptide according claim 1 or 14 comprising:
a) contacting a polypeptide according to claim 1 or 14 with a candidate compound and
b) determining whether compound binds to said polypeptide.

22. A method for identifying compounds which have a stimulatory or inhibitory effect on the biological activity of a polypeptide according claim 1 or 14 comprising
a) contacting a polypeptide according to claim 1 or 14 with a candidate compound and
b) determining if said compound has modulated the function or activity of said polypeptide.


SEQUENCE LISTING

<110>  F. Hoffmann-La Roche

<120>  Human Trace Amine Associated Receptors

<130>  22645

<160>  36

<170>  PatentIn version 3.2

<210>  1
<211>  16
<212>  PRT
<213>  Homo sapiens

<220>
<221>  fingerprint
<222>  (1)..(16)

<220>
<221>  misc_feature
<222>  (3)..(4)
<223>  Xaa can be any naturally occurring amino acid

<220>
<221>  misc_feature
<222>  (7)..(8)
<223>  Xaa can be any naturally occurring amino acid

<220>
<221>  misc_feature
<222>  (10)..(12)
<223>  Xaa can be any naturally occurring amino acid

<220>
<221>  misc_feature
<222>  (14)..(14)
<223>  Xaa can be any naturally occurring amino acid

<400>  1

Asn Ser Xaa Xaa Asn Pro Xaa Xaa Tyr Xaa Xaa Xaa Tyr Xaa Trp Phe
1               5                   10                  15

<210>  2
<211>  1020
<212>  DNA
<213>  Homo sapiens

<220>
<221>  huTAAR1
<222>  (1)..(1020)

<400>  2
atgatgccct tttgccacaa tataattaat atttcctgtg tgaaaaacaa ctggtcaaat
60

gatgtccgtg cttccctgta cagtttaatg gtgctcataa ttctgaccac actcgttggc
120

aatctgatag ttattgtttc tatatcacac ttcaaacaac ttcatacccc aacaaattgg
180

ctcattcatt ccatggccac tgtggacttt cttctggggt gtctggtcat gccttacagt
240

atggtgagat ctgctgagca ctgttggtat tttggagaag tcttctgtaa aattcacaca
300

agcaccgaca ttatgctgag ctcagcctcc attttccatt tgtctttcat ctccattgac
360

cgctactatg ctgtgtgtga tccactgaga tataaagcca agatgaatat cttggttatt
420

tgtgtgatga tcttcattag ttggagtgtc cctgctgttt ttgcatttgg aatgatcttt
480

ctggagctaa acttcaaagg cgctgaagag atatattaca aacatgttca ctgcagagga
540

ggttgctctg tcttctttag caaaatatct ggggtactga cctttatgac ttctttttat
600

atacctggat ctattatgtt atgtgtctat tacagaatat atcttatcgc taaagaacag
660

gcaagattaa ttagtgatgc caatcagaag ctccaaattg gattggaaat gaaaaatgga
720

atttcacaaa gcaaagaaag gaaagctgtg aagacattgg ggattgtgat gggagttttc
780

ctaatatgct ggtgcccttt ctttatctgt acagtcatgg acccttttct tcactacatt
840

attccaccta ctttgaatga tgtattgatt tggtttggct acttgaactc tacatttaat
900

ccaatggttt atgcattttt ctatccttgg tttagaaaag cactgaagat gatgctgttt
960

ggtaaaattt tccaaaaaga ttcatccagg tgtaaattat ttttggaatt gagttcataa
1020


<210>  3
<211>  339
<212>  PRT
<213>  Homo sapiens


<220>
<221>  huTAAR 1
<222>  (1)..(281)

<400>  3

Met Met Pro Phe Cys His Asn Ile Ile Asn Ile Ser Cys Val Lys Asn
1               5                   10                  15


Asn Trp Ser Asn Asp Val Arg Ala Ser Leu Tyr Ser Leu Met Val Leu
            20                  25                  30


Ile Ile Leu Thr Thr Leu Val Gly Asn Leu Ile Val Ile Val Ser Ile
        35                  40                  45


Ser His Phe Lys Gln Leu His Thr Pro Thr Asn Trp Leu Ile His Ser
    50                  55                  60

Met Ala Thr Val Asp Phe Leu Leu Gly Cys Leu Val Met Pro Tyr Ser
65                  70                  75                  80

Met Val Arg Ser Ala Glu His Cys Trp Tyr Phe Gly Glu Val Phe Cys
                85                  90                  95

Lys Ile His Thr Ser Thr Asp Ile Met Leu Ser Ser Ala Ser Ile Phe
            100                 105                 110

His Leu Ser Phe Ile Ser Ile Asp Arg Tyr Tyr Ala Val Cys Asp Pro
        115                 120                 125

Leu Arg Tyr Lys Ala Lys Met Asn Ile Leu Val Ile Cys Val Met Ile
    130                 135                 140

Phe Ile Ser Trp Ser Val Pro Ala Val Phe Ala Phe Gly Met Ile Phe
145                 150                 155                 160

Leu Glu Leu Asn Phe Lys Gly Ala Glu Glu Ile Tyr Tyr Lys His Val
                165                 170                 175

His Cys Arg Gly Gly Cys Ser Val Phe Phe Ser Lys Ile Ser Gly Val
            180                 185                 190

Leu Thr Phe Met Thr Ser Phe Tyr Ile Pro Gly Ser Ile Met Leu Cys
        195                 200                 205

Val Tyr Tyr Arg Ile Tyr Leu Ile Ala Lys Glu Gln Ala Arg Leu Ile
    210                 215                 220

Ser Asp Ala Asn Gln Lys Leu Gln Ile Gly Leu Glu Met Lys Asn Gly
225                 230                 235                 240

Ile Ser Gln Ser Lys Glu Arg Lys Ala Val Lys Thr Leu Gly Ile Val
                245                 250                 255

Met Gly Val Phe Leu Ile Cys Trp Cys Pro Phe Phe Ile Cys Thr Val
            260                 265                 270

Met Asp Pro Phe Leu His Tyr Ile Ile Pro Pro Thr Leu Asn Asp Val
        275                 280                 285

Leu Ile Trp Phe Gly Tyr Leu Asn Ser Thr Phe Asn Pro Met Val Tyr
    290                 295                 300

Ala Phe Phe Tyr Pro Trp Phe Arg Lys Ala Leu Lys Met Met Leu Phe
305                 310                 315                 320

Gly Lys Ile Phe Gln Lys Asp Ser Ser Arg Cys Lys Leu Phe Leu Glu
                325                 330                 335

Leu Ser Ser

<210> 4
<211> 1056
<212> DNA
<213> Homo sapiens

<220>
<221> huTAAR 2
<222> (1)..(1056)

<400> 4
atggctgtct catcagagca acatgaactt tcacatttca aaagaacaca gacaaaaaag
60

gaaaaattca attgctctga atatggaaat agatcttgcc cagaaaatga aagatctctg
120

ggtgtccgag tggctatgta ttcatttatg gcaggatcca tattcatcac aatatttggc
180

aatcttgcca tgataatttc catttcctac ttcaagcagc ttcacacacc aaccaacttc
240

ctcatcctct ccatggccat cactgatttc ctcctgggat tcaccatcat gccatatagt
300

atgatcagat cggtggagaa ctgctggtat tttgggctta cattttgcaa gatttattat
360

agttttgacc tgatgcttag cataacatcc atttttcatc tttgctcagt ggccattgat
420

agattttatg ctatatgtta cccattactt tattccacca aaataactat tccagtcatt
480

aaaagattgc tacttctatg ttggtcggtc cctggagcat ttgccttcgg ggtggtcttc
540

tcagaggcct atgcagatgg aatagagggc tatgacatct tggttgcttg ttccagttcc
600

tgcccagtga tgttcaacaa gctatggggg accaccttgt ttatggcagg tttcttcact
660

cctgggtcta tgatggtggg gatttatggc aaaatttttg cagtatccag aaaacatgct
720

catgccatca ataacttgcg agaaaatcaa aataatcaag tgaagaaaga caaaaaagct
780

gccaaaactt taggaatagt gataggagtt ttcttattat gttggtttcc ttgtttcttc
840

acaattttat tggatccctt tttgaacttc tctactcctg tagttttgtt tgatgccttg
900

acatggtttg gctattttaa ctccacatgt aatccgttaa tatatggttt cttctatccc
960

tggtttcgca gagcactgaa gtacattttg ctaggtaaaa ttttcagctc atgtttccat
1020

aatactattt tgtgtatgca aaaagaaagt gagtag
1056

<210> 5

<211>  351
<212>  PRT
<213>  Homo sapiens

<220>
<221>  huTAAR 2
<222>  (1)..(351)

<400>  5

Met Ala Val Ser Ser Glu Gln His Glu Leu Ser His Phe Lys Arg Thr
1               5                   10                  15

Gln Thr Lys Lys Glu Lys Phe Asn Cys Ser Glu Tyr Gly Asn Arg Ser
            20                  25                  30

Cys Pro Glu Asn Glu Arg Ser Leu Gly Val Arg Val Ala Met Tyr Ser
        35                  40                  45

Phe Met Ala Gly Ser Ile Phe Ile Thr Ile Phe Gly Asn Leu Ala Met
    50                  55                  60

Ile Ile Ser Ile Ser Tyr Phe Lys Gln Leu His Thr Pro Thr Asn Phe
65                  70                  75                  80

Leu Ile Leu Ser Met Ala Ile Thr Asp Phe Leu Leu Gly Phe Thr Ile
                85                  90                  95

Met Pro Tyr Ser Met Ile Arg Ser Val Glu Asn Cys Trp Tyr Phe Gly
            100                 105                 110

Leu Thr Phe Cys Lys Ile Tyr Tyr Ser Phe Asp Leu Met Leu Ser Ile
        115                 120                 125

Thr Ser Ile Phe His Leu Cys Ser Val Ala Ile Asp Arg Phe Tyr Ala
    130                 135                 140

Ile Cys Tyr Pro Leu Leu Tyr Ser Thr Lys Ile Thr Ile Pro Val Ile
145                 150                 155                 160

Lys Arg Leu Leu Leu Leu Cys Trp Ser Val Pro Gly Ala Phe Ala Phe
                165                 170                 175

Gly Val Val Phe Ser Glu Ala Tyr Ala Asp Gly Ile Glu Gly Tyr Asp
            180                 185                 190

Ile Leu Val Ala Cys Ser Ser Ser Cys Pro Val Met Phe Asn Lys Leu
        195                 200                 205

Trp Gly Thr Thr Leu Phe Met Ala Gly Phe Phe Thr Pro Gly Ser Met
    210                 215                 220

Met Val Gly Ile Tyr Gly Lys Ile Phe Ala Val Ser Arg Lys His Ala
225                 230                 235                 240

His Ala Ile Asn Asn Leu Arg Glu Asn Gln Asn Asn Gln Val Lys Lys
                245                 250                 255

Asp Lys Lys Ala Ala Lys Thr Leu Gly Ile Val Ile Gly Val Phe Leu
            260                 265                 270

Leu Cys Trp Phe Pro Cys Phe Phe Thr Ile Leu Leu Asp Pro Phe Leu
        275                 280                 285

Asn Phe Ser Thr Pro Val Val Leu Phe Asp Ala Leu Thr Trp Phe Gly
    290                 295                 300

Tyr Phe Asn Ser Thr Cys Asn Pro Leu Ile Tyr Gly Phe Phe Tyr Pro
305                 310                 315                 320

Trp Phe Arg Arg Ala Leu Lys Tyr Ile Leu Leu Gly Lys Ile Phe Ser
                325                 330                 335

Ser Cys Phe His Asn Thr Ile Leu Cys Met Gln Lys Glu Ser Glu
            340                 345                 350

<210>  6
<211>  1030
<212>  DNA
<213>  Homo sapiens

<220>
<221>  huTAAR 3 Y
<222>  (1)..(1030)
<223>  Pseudogene

<400>  6
atggatctaa cttatattcc cgaagaccta tccagttgtc caaaatttgt aaataaatcc
60

tgtcctccca ccaaccgctc ttttcatgtc caggtgataa tgtattcggt tatgactgga
120

gccatgatta tcactattcg gaaacttggt tataatggtt tccatatcgc atttcaaaca
180

gcttcactct cccacaaact ttctgatcct ctccatggca accacggact ttctgctggg
240

ttttgtcatt atgccataca gcataatgcg atcagtggag agttgctggt actttgggga
300

tggcttttgt aaattccaca caagctttga catgatgctc agactgacct ccattttcca
360

cctctgttcc attgctattg accgatttta tgccgtgtgt taccctttac attacacaac
420

caaaatgacg aactccacca taaagcaact gctggcattt tgctggtcag ttcctgctct
480

tttttctttt ggtttagttc tatctgaggc cgatgtttcc ggtatgcaga gctataagat
540

acttgttgct tgcttcaatt tctgtgccct tactttcaac aaattctggg ggacaatatt
600

gttcactaca tgtttcttta cccctggctc catcatggtt ggtatttatg gcaaaatctt
660

tatcgtttcc aaacagcatg ctcgagtcat cagccatgtg cctgaaaaca caaagggggc
720

agtgaaaaaa cacctatcca agaaaaagga caggaaagca gcgaagacac tgggtatagt
780

aatgggggtg tttctggctt gctggttgcc ttgttttctt gctgttctga ttgacccata
840

cctagactac tccactccca tactaatatt ggatctttta gtgtggctcc ggtacttcaa
900

ctctacttgc aaccctctta ttcatggctt ttttaatcca tggtttcaga aagcattcaa
960

gtacatagtg tcaggaaaaa tatttagctc ccattcagaa actgcaaatt tgtttcctga
1020

agcacattaa
1030

<210>   7
<211>   1032
<212>   DNA
<213>   Homo sapiens

<220>
<221>   huTAAR 3 Y fixed
<222>   (1)..(1032)
<223>   repaired pseudogene

<400>   7
   atggatctaa cttatattcc cgaagaccta tccagttgtc caaaatttgt aaataaatcc
   60
   tgtcctccca ccaaccgctc ttttcatgtc caggtgataa tgtattcggt tatgactgga
   120
   gccatgatta tcaccatatt cggaaacttg gttataatgg tttccatatc gcatttcaaa
   180
   cagcttcact ctcccacaaa ctttctgatc ctctccatgg caaccacgga ctttctgctg
   240
   ggttttgtca ttatgccata cagcataatg cgatcagtgg agagttgctg gtactttggg
   300
   gatggctttt gtaaattcca cacaagcttt gacatgatgc tcagactgac ctccattttc
   360
   cacctctgtt ccattgctat tgaccgattt tatgccgtgt gttacccttt acattacaca
   420
   accaaaatga cgaactccac cataaagcaa ctgctggcat tttgctggtc agttcctgct
   480
   ctttttcttt ttggtttagt tctatctgag gccgatgttt ccggtatgca gagctataag
   540
   atacttgttg cttgcttcaa tttctgtgcc cttactttca acaaattctg ggggacaata
   600
   ttgttcacta catgtttctt tacccctggc tccatcatgg ttggtattta tggcaaaatc
   660
   tttatcgttt ccaaacagca tgctcgagtc atcagccatg tgcctgaaaa cacaaagggg
   720
   gcagtgaaaa aacacctatc caagaaaaag gacaggaaag cagcgaagac actgggtata
   780
   gtaatggggg tgtttctggc ttgctggttg ccttgttttc ttgctgttct gattgaccca
   840
   tacctagact actccactcc catactaata ttggatcttt tagtgtggct ccggtacttc
   900
   aactctactt gcaaccctct tattcatggc ttttttaatc catggtttca gaaagcattc
   960
   aagtacatag tgtcaggaaa aatatttagc tcccattcag aaactgcaaa tttgtttcct
   1020
   gaagcacatt aa
   1032

<210>  8
<211>  343
<212>  PRT
<213>  Homo sapiens

<220>
<221>  huTAAR 3 Y fixed
<222>  (1)..(343)
<223>  amino acid sequence of repaired pseudogene

<400>  8

Met Asp Leu Thr Tyr Ile Pro Glu Asp Leu Ser Ser Cys Pro Lys Phe
1               5                   10                  15

Val Asn Lys Ser Cys Pro Pro Thr Asn Arg Ser Phe His Val Gln Val
            20                  25                  30

Ile Met Tyr Ser Val Met Thr Gly Ala Met Ile Ile Thr Ile Phe Gly
        35                  40                  45

Asn Leu Val Ile Met Val Ser Ile Ser His Phe Lys Gln Leu His Ser
    50                  55                  60

Pro Thr Asn Phe Leu Ile Leu Ser Met Ala Thr Thr Asp Phe Leu Leu
65                  70                  75                  80

Gly Phe Val Ile Met Pro Tyr Ser Ile Met Arg Ser Val Glu Ser Cys
                85                  90                  95

Trp Tyr Phe Gly Asp Gly Phe Cys Lys Phe His Thr Ser Phe Asp Met
            100                 105                 110

Met Leu Arg Leu Thr Ser Ile Phe His Leu Cys Ser Ile Ala Ile Asp
        115                 120                 125

Arg Phe Tyr Ala Val Cys Tyr Pro Leu His Tyr Thr Thr Lys Met Thr
    130                 135                 140

Asn Ser Thr Ile Lys Gln Leu Leu Ala Phe Cys Trp Ser Val Pro Ala
145                 150                 155                 160

Leu Phe Ser Phe Gly Leu Val Leu Ser Glu Ala Asp Val Ser Gly Met
                165                 170                 175

Gln Ser Tyr Lys Ile Leu Val Ala Cys Phe Asn Phe Cys Ala Leu Thr
            180                 185                 190

Phe Asn Lys Phe Trp Gly Thr Ile Leu Phe Thr Thr Cys Phe Phe Thr
        195                 200                 205

Pro Gly Ser Ile Met Val Gly Ile Tyr Gly Lys Ile Phe Ile Val Ser
    210                 215                 220

Lys Gln His Ala Arg Val Ile Ser His Val Pro Glu Asn Thr Lys Gly
225                 230                 235                 240

Ala Val Lys Lys His Leu Ser Lys Lys Lys Asp Arg Lys Ala Ala Lys
                245                 250                 255

Thr Leu Gly Ile Val Met Gly Val Phe Leu Ala Cys Trp Leu Pro Cys
            260                 265                 270

Phe Leu Ala Val Leu Ile Asp Pro Tyr Leu Asp Tyr Ser Thr Pro Ile
        275                 280                 285

Leu Ile Leu Asp Leu Leu Val Trp Leu Arg Tyr Phe Asn Ser Thr Cys
    290                 295                 300

Asn Pro Leu Ile His Gly Phe Phe Asn Pro Trp Phe Gln Lys Ala Phe
305                 310                 315                 320

Lys Tyr Ile Val Ser Gly Lys Ile Phe Ser Ser His Ser Glu Thr Ala
                325                 330                 335

Asn Leu Phe Pro Glu Ala His
            340

<210>  9
<211>  1049
<212>  DNA
<213>  Homo sapiens

<220>
<221>  huTAAR 4 Y
<222>  (1)..(1049)
<223>  Pseudogene

discrepancy to U88828: early stop codon

<400>  9
atgatgaatt tgcctgaccc tcagaacccc ccaacagtac aattttgctt tagttcagtt
60

aacaattcat gccctagaaa tgtgaggccg gtgctgagtg tctgggccat gtacctggtc
120

atgatcgggt ctatagtgat gacaatgctg ggcaacatga tcgtaatgat ttccatcgct
180

cacttcaagc agctccactc cccgaccaac ttcttgatcc tctccatggc catcactgac
240

tttttgctga gctgtgtggt catgcccttc agtgtgatca catccatcga gtcctgctgg
300

tattttggag acctcttttg caaagtccac agctgctgtg acatcatact ctgcaccacc
360

tccatttttc acctctgctt catctcagtt gaccgttacg atgctgtttg agacccattg
420

caatatgtca ccagaattac catccctgtc atagaactct ttctactcat cagttggtcc
480

attcccatcc tttttgcctt tggcctggta ttctcaaaac taaacataat tggtgcagaa
540

gagtttgttg cagccattga ttgcacaggt ttgtgtgtgt taatatttaa taagctctgg
600

gggggtactg gcctccttta tagctttctt tctcccaggg acaaccacgg tgggaattta
660

catacatatt tttacagtag ccaggaagca tgccatgcaa attggcacag gttctaggac
720

taaacaggct gggtcagaaa gcaaaaaaaa aggcatcctc taaaacagaa agcaaggcca
780

ccaggacctt aggcatagtc atgggagtgt ttgtgttgtg ctggctgccc ttctttgtct
840

tgacgatcac agatcctttc attaatttta caacccttga agatctgtac aatgtcttcc
900

tctggctagg ctatttcaac tctgctttca atcccatttt atatggcatg ctttatcctt
960

ggtttcgcaa ggcattgagg atgattgtca caggcatgat cttccaccct gactcttcca
1020

ccctaagcct gttttctgcc catgcttag
1049

<210> 10
<211> 1047
<212> DNA
<213> Homo sapiens

<220>
<221> huTAAR 4 Y fixed
<222> (1)..(1047)
<223> repaired pseudogene

<400> 10
atgatgaatt tgcctgaccc tcagaacccc ccaacagtac aattttgctt tagttcagtt
60

aacaattcat gccctagaaa tgtgaggccg gtgctgagtg tctgggccat gtacctggtc
120

atgatcgggt ctatagtgat gacaatgctg ggcaacatga tcgtaatgat ttccatcgct
180

cacttcaagc agctccactc cccgaccaac ttcttgatcc tctccatggc catcactgac
240

tttttgctga gctgtgtggt catgcccttc agtgtgatca catccatcga gtcctgctgg
300

tattttggag acctcttttg caaagtccac agctgctgtg acatcatact ctgcaccacc
360

tccatttttc acctctgctt catctcagtt gaccgttacg atgctgtttg tgacccattg
420

caatatgtca ccagaattac catccctgtc atagaactct ttctactcat cagttggtcc
480

attcccatcc tttttgcctt tggcctggta ttctcaaaac taaacataat tggtgcagaa
540

gagtttgttg cagccattga ttgcacaggt ttgtgtgtgt taatatttaa taagctctgg
600

ggggtactgg cctcctttat agctttcttt ctcccaggga caaccacggt gggaatttac
660

atacatattt ttacagtagc caggaagcat gccatgcaaa ttggcacagg ttctaggact
720

aaacaggctg ggtcagaaag caaaaaaaag gcatcctcta aaacagaaag caaggccacc
780

aggaccttag gcatagtcat gggagtgttt gtgttgtgct ggctgccctt ctttgtcttg
840

acgatcacag atcctttcat taattttaca acccttgaag atctgtacaa tgtcttcctc
900

tggctaggct atttcaactc tgctttcaat cccattttat atggcatgct ttatccttgg
960

tttcgcaagg cattgaggat gattgtcaca ggcatgatct tccaccctga ctcttccacc
1020

ctaagcctgt tttctgccca tgcttag
1047


<210> 11
<211> 348
<212> PRT
<213> Homo sapiens


<220>
<221> huTAAR 4 Y fixed
<222> (1)..(348)
<223> amino acid sequence of repaired pseudogene

<400> 11

Met Met Asn Leu Pro Asp Pro Gln Asn Pro Pro Thr Val Gln Phe Cys
1               5                   10                  15


Phe Ser Ser Val Asn Asn Ser Cys Pro Arg Asn Val Arg Pro Val Leu
            20                  25                  30

Ser Val Trp Ala Met Tyr Leu Val Met Ile Gly Ser Ile Val Met Thr
        35                  40                  45

Met Leu Gly Asn Met Ile Val Met Ile Ser Ile Ala His Phe Lys Gln
    50                  55                  60

Leu His Ser Pro Thr Asn Phe Leu Ile Leu Ser Met Ala Ile Thr Asp
65                  70                  75                  80

Phe Leu Leu Ser Cys Val Val Met Pro Phe Ser Val Ile Thr Ser Ile
                85                  90                  95

Glu Ser Cys Trp Tyr Phe Gly Asp Leu Phe Cys Lys Val His Ser Cys
            100                 105                 110

Cys Asp Ile Ile Leu Cys Thr Thr Ser Ile Phe His Leu Cys Phe Ile
        115                 120                 125

Ser Val Asp Arg Tyr Asp Ala Val Cys Asp Pro Leu Gln Tyr Val Thr
    130                 135                 140

Arg Ile Thr Ile Pro Val Ile Glu Leu Phe Leu Leu Ile Ser Trp Ser
145                 150                 155                 160

Ile Pro Ile Leu Phe Ala Phe Gly Leu Val Phe Ser Lys Leu Asn Ile
                165                 170                 175

Ile Gly Ala Glu Glu Phe Val Ala Ala Ile Asp Cys Thr Gly Leu Cys
            180                 185                 190

Val Leu Ile Phe Asn Lys Leu Trp Gly Val Leu Ala Ser Phe Ile Ala
        195                 200                 205

Phe Phe Leu Pro Gly Thr Thr Thr Val Gly Ile Tyr Ile His Ile Phe
    210                 215                 220

Thr Val Ala Arg Lys His Ala Met Gln Ile Gly Thr Gly Ser Arg Thr
225                 230                 235                 240

Lys Gln Ala Gly Ser Glu Ser Lys Lys Lys Ala Ser Ser Lys Thr Glu
                245                 250                 255

Ser Lys Ala Thr Arg Thr Leu Gly Ile Val Met Gly Val Phe Val Leu
            260                 265                 270

Cys Trp Leu Pro Phe Phe Val Leu Thr Ile Thr Asp Pro Phe Ile Asn
        275                 280                 285

Phe Thr Thr Leu Glu Asp Leu Tyr Asn Val Phe Leu Trp Leu Gly Tyr
    290                 295                 300

Phe Asn Ser Ala Phe Asn Pro Ile Leu Tyr Gly Met Leu Tyr Pro Trp

305                 310                 315                 320

Phe Arg Lys Ala Leu Arg Met Ile Val Thr Gly Met Ile Phe His Pro
                325                 330                 335

Asp Ser Ser Thr Leu Ser Leu Phe Ser Ala His Ala
            340                 345

<210>  12
<211>  1014
<212>  DNA
<213>  Homo sapiens

<220>
<221>  huTAAR 5
<222>  (1)..(1014)

<400>  12
atgagagctg tcttcatcca aggtgctgaa gagcaccctg cggcattctg ctaccaggtg
60

aatgggtctt gccccaggac agtacatact ctgggcatcc agttggtcat ctacctggcc
120

tgtgcagcag gcatgctgat tatcgtgcta gggaatgtat ttgtggcatt tgctgtgtcc
180

tacttcaaag cgcttcacac gcccaccaac ttcctgctgc tctccctggc cctggctgac
240

atgtttctgg gtctgctggt gctgcccctc agcaccattc gctcagtgga gagctgctgg
300

ttcttcgggg acttcctctg ccgcctgcac acctacctgg acaccctctt ctgcctcacc
360

tccatcttcc atctctgttt catttccatt gaccgccact gtgccatctg tgaccccctg
420

ctctatccct ccaagttcac agtgagggtg gctctcaggt acatcctggc aggatggggg
480

gtgcccgcag catacacttc gttattcctc tacacagatg tggtagagac aaggctcagc
540

cagtggctgg aagagatgcc ttgtgtgggc agttgccagc tgctgctcaa taaattttgg
600

ggctggttaa acttcccttt gttctttgtc ccctgcctca ttatgatcag cttgtatgtg
660

aagatctttg tggttgctac cagacaggct cagcagatta ccacattgag caaaagcctg
720

gctggggctg ccaagcatga gagaaaagct gccaagaccc tgggcattgc tgtgggcata
780

tacctcttgt gctggctgcc cttcaccata gacacgatgg tcgacagcct ccttcacttt
840

atcacacccc cactggtctt tgacatcttt atctggtttg cttacttcaa ctcagcctgc
900

aaccccatca tctatgtctt ttcctaccag tggtttcgga aggcactgaa actcacactg
960

agccagaagg tcttctcacc gcagacacgc actgttgatt tgtaccaaga atga
1014

<210> 13
<211> 337
<212> PRT
<213> Homo sapiens

<220>
<221> huTAAR 5
<222> (1)..(337)

<400> 13

Met Arg Ala Val Phe Ile Gln Gly Ala Glu Glu His Pro Ala Ala Phe
1               5                   10                  15

Cys Tyr Gln Val Asn Gly Ser Cys Pro Arg Thr Val His Thr Leu Gly
            20                  25                  30

Ile Gln Leu Val Ile Tyr Leu Ala Cys Ala Ala Gly Met Leu Ile Ile
        35                  40                  45

Val Leu Gly Asn Val Phe Val Ala Phe Ala Val Ser Tyr Phe Lys Ala
    50                  55                  60

Leu His Thr Pro Thr Asn Phe Leu Leu Leu Ser Leu Ala Leu Ala Asp
65                  70                  75                  80

Met Phe Leu Gly Leu Leu Val Leu Pro Leu Ser Thr Ile Arg Ser Val
                85                  90                  95

Glu Ser Cys Trp Phe Phe Gly Asp Phe Leu Cys Arg Leu His Thr Tyr
            100                 105                 110

Leu Asp Thr Leu Phe Cys Leu Thr Ser Ile Phe His Leu Cys Phe Ile
        115                 120                 125

Ser Ile Asp Arg His Cys Ala Ile Cys Asp Pro Leu Leu Tyr Pro Ser
    130                 135                 140

Lys Phe Thr Val Arg Val Ala Leu Arg Tyr Ile Leu Ala Gly Trp Gly
145                 150                 155                 160

Val Pro Ala Ala Tyr Thr Ser Leu Phe Leu Tyr Thr Asp Val Val Glu
                165                 170                 175

Thr Arg Leu Ser Gln Trp Leu Glu Glu Met Pro Cys Val Gly Ser Cys
            180                 185                 190

Gln Leu Leu Leu Asn Lys Phe Trp Gly Trp Leu Asn Phe Pro Leu Phe
        195                 200                 205

Phe Val Pro Cys Leu Ile Met Ile Ser Leu Tyr Val Lys Ile Phe Val
    210                 215                 220

Val Ala Thr Arg Gln Ala Gln Gln Ile Thr Thr Leu Ser Lys Ser Leu
225                 230                 235                 240

Ala Gly Ala Ala Lys His Glu Arg Lys Ala Ala Lys Thr Leu Gly Ile
                245                 250                 255

Ala Val Gly Ile Tyr Leu Leu Cys Trp Leu Pro Phe Thr Ile Asp Thr
            260                 265                 270

Met Val Asp Ser Leu Leu His Phe Ile Thr Pro Pro Leu Val Phe Asp
        275                 280                 285

Ile Phe Ile Trp Phe Ala Tyr Phe Asn Ser Ala Cys Asn Pro Ile Ile
    290                 295                 300

Tyr Val Phe Ser Tyr Gln Trp Phe Arg Lys Ala Leu Lys Leu Thr Leu
305                 310                 315                 320

Ser Gln Lys Val Phe Ser Pro Gln Thr Arg Thr Val Asp Leu Tyr Gln
                325                 330                 335

Glu

<210>  14
<211>  1038
<212>  DNA
<213>  Homo sapiens

<220>
<221>  huTAAR 6
<222>  (1)..(1038)
<223>  representative cDNA (AF389192 as of 03.06.2004),

       for gene under UniGene ID Hs.434196

<400>  14
atgagcagca attcatccct gctggtggct gtgcagctgt gctacgcgaa cgtgaatggg
60

tcctgtgtga aaatcccctt ctcgccggga tcccgggtga ttctgtacat agtgtttggc
120

tttggggctg tgctggctgt gtttggaaac ctcctggtga tgatttcaat cctccatttc
180

aagcagctgc actctccgac caattttctc gttgcctctc tggcctgcgc tgatttcttg
240

gtgggtgtga ctgtgatgcc cttcagcatg gtcaggacgg tggagagctg ctggtatttt
300

gggaggagtt tttgtacttt ccacacctgc tgtgatgtgg cattttgtta ctcttctctc
360

tttcacttgt gcttcatctc catcgacagg tacattgcgg ttactgaccc cctggtctat
420

cctaccaagt tcaccgtatc tgtgtcagga atttgcatca gcgtgtcctg gatcctgccc
480

ctcatgtaca gcggtgctgt gttctacaca ggtgtctatg acgatgggct ggaggaatta
540

tctgatgccc taaactgtat aggaggttgt cagaccgttg taaatcaaaa ctgggtgttg
600

acagattttc tatccttctt tatacctacc tttattatga taattctgta tggtaacata
660

tttcttgtgg ctagacgaca ggcgaaaaag atagaaaata ctggtagcaa gacagaatca
720

tcctcagaga gttacaaagc cagagtggcc aggagagaga gaaaagcagc taaaaccctg
780

ggggtcacag tggtagcatt tatgatttca tggttaccat atagcattga ttcattaatt
840

gatgccttta tgggctttat aacccctgcc tgtatttatg agatttgctg ttggtgtgct
900

tattataact cagccatgaa tcctttgatt tatgctttat tttacccatg gtttaggaaa
960

gcaataaaag ttattgtaac tggtcaggtt ttaaagaaca gttcagcaac catgaatttg
1020

ttttctgaac atatataa
1038

<210> 15
<211> 345
<212> PRT
<213> Homo sapiens

<220>
<221> huTAAR 6
<222> (1)..(345)

<400> 15

Met Ser Ser Asn Ser Ser Leu Leu Val Ala Val Gln Leu Cys Tyr Ala
1               5                   10                  15

Asn Val Asn Gly Ser Cys Val Lys Ile Pro Phe Ser Pro Gly Ser Arg
            20                  25                  30

Val Ile Leu Tyr Ile Val Phe Gly Phe Gly Ala Val Leu Ala Val Phe
        35                  40                  45

Gly Asn Leu Leu Val Met Ile Ser Ile Leu His Phe Lys Gln Leu His
    50                  55                  60

Ser Pro Thr Asn Phe Leu Val Ala Ser Leu Ala Cys Ala Asp Phe Leu
65                  70                  75                  80

Val Gly Val Thr Val Met Pro Phe Ser Met Val Arg Thr Val Glu Ser
                85                  90                  95

Cys Trp Tyr Phe Gly Arg Ser Phe Cys Thr Phe His Thr Cys Cys Asp
            100                 105                 110

Val Ala Phe Cys Tyr Ser Ser Leu Phe His Leu Cys Phe Ile Ser Ile
        115                 120                 125

Asp Arg Tyr Ile Ala Val Thr Asp Pro Leu Val Tyr Pro Thr Lys Phe
    130                 135                 140

Thr Val Ser Val Ser Gly Ile Cys Ile Ser Val Ser Trp Ile Leu Pro
145                 150                 155                 160

Leu Met Tyr Ser Gly Ala Val Phe Tyr Thr Gly Val Tyr Asp Asp Gly
                165                 170                 175

Leu Glu Glu Leu Ser Asp Ala Leu Asn Cys Ile Gly Gly Cys Gln Thr
            180                 185                 190

Val Val Asn Gln Asn Trp Val Leu Thr Asp Phe Leu Ser Phe Phe Ile
        195                 200                 205

Pro Thr Phe Ile Met Ile Ile Leu Tyr Gly Asn Ile Phe Leu Val Ala
    210                 215                 220

Arg Arg Gln Ala Lys Lys Ile Glu Asn Thr Gly Ser Lys Thr Glu Ser
225                 230                 235                 240

Ser Ser Glu Ser Tyr Lys Ala Arg Val Ala Arg Arg Glu Arg Lys Ala
                245                 250                 255

Ala Lys Thr Leu Gly Val Thr Val Val Ala Phe Met Ile Ser Trp Leu
            260                 265                 270

Pro Tyr Ser Ile Asp Ser Leu Ile Asp Ala Phe Met Gly Phe Ile Thr
        275                 280                 285

Pro Ala Cys Ile Tyr Glu Ile Cys Cys Trp Cys Ala Tyr Tyr Asn Ser
    290                 295                 300

Ala Met Asn Pro Leu Ile Tyr Ala Leu Phe Tyr Pro Trp Phe Arg Lys
305                 310                 315                 320

Ala Ile Lys Val Ile Val Thr Gly Gln Val Leu Lys Asn Ser Ser Ala
                325                 330                 335

Thr Met Asn Leu Phe Ser Glu His Ile
            340                 345

<210>  16
<211>  210
<212>  DNA
<213>  Homo sapiens

<220>
<221>  huTAAR 7 Y fragment
<222>  (1)..(210)

<400>  16
gtggtagcat tttaaacctc atggttacta tatatgatga ttgattcatt cgttggttct
60

ctttggggtt ttatcatacc ttcctatgtt tatgagatat tttcctagtt tggttactct
120

aactgtgttc caaatacttt gatttacgct ttattttacc cttggcttag gggtgcaata
180

aaacttatta taactggaca atatgaaaag
210


<210>  17
<211>  1029
<212>  DNA
<213>  Homo sapiens


<220>
<221>  huTAAR 8
<222>  (1)..(1029)
<223>  representative cDNA (AF380193 as of 03.06.2004),

       of gene of UniGene ID Hs.350571

<400>  17
atgaccagca atttttccca acctgttgtg cagctttgct atgaggatgt gaatggatct
60

tgtattgaaa ctccctattc tcctgggtcc cgggtaattc tgtacacggc gtttagcttt
120

gggtctttgc tggctgtatt tggaaatctc ttagtaatga cttctgttct tcattttaag
180

cagctgcact ctccaaccaa ttttctcatt gcctctctgg cctgtgctga cttcttggta
240

ggtgtgactg tgatgctttt cagcatggtc aggacggtgg agagctgctg gtattttgga
300

gccaaatttt gtactcttca cagttgctgt gatgtggcat tttgttactc ttctgtcctc
360

cacttgtgct tcatctgcat cgacaggtac attgtggtta ctgatcccct ggtctatgct
420

accaagttca ccgtgtctgt gtcgggaatt tgcatcagcg tgtcctggat tctgcctctc
480

acgtacagcg gtgctgtgtt ctacacaggt gtcaatgatg atgggctgga ggaattagta
540

agtgctctca actgcgtagg tggctgtcaa attattgtaa gtcaaggctg ggtgttgata
600

gattttctgt tattcttcat acctaccctt gttatgataa ttctttacag taagattttt
660

cttatagcta aacaacaagc tataaaaatt gaaactacta gtagcaaagt agaatcatcc
720

tcagagagtt ataaaatcag agtggccaag agagagagga aagcagctaa aaccctgggg
780

gtcacggtac tagcatttgt tatttcatgg ttaccgtata cagttgatat attaattgat
840

gcctttatgg gcttcctgac ccctgcctat atctatgaaa tttgctgttg gagtgcttat
900

tataactcag ccatgaatcc tttgatttat gctctatttt atccttggtt taggaaagcc
960

ataaaactta ttttaagtgg agatgtttta aaggctagtt catcaaccat tagtttattt
1020

ttagaataa
1029

<210> 18
<211> 342
<212> PRT
<213> Homo sapiens

<220>
<221> huTAAR 8
<222> (1)..(342)

<400> 18

Met Thr Ser Asn Phe Ser Gln Pro Val Val Gln Leu Cys Tyr Glu Asp
1               5                   10                  15

Val Asn Gly Ser Cys Ile Glu Thr Pro Tyr Ser Pro Gly Ser Arg Val
            20                  25                  30

Ile Leu Tyr Thr Ala Phe Ser Phe Gly Ser Leu Leu Ala Val Phe Gly
        35                  40                  45

Asn Leu Leu Val Met Thr Ser Val Leu His Phe Lys Gln Leu His Ser
    50                  55                  60

Pro Thr Asn Phe Leu Ile Ala Ser Leu Ala Cys Ala Asp Phe Leu Val
65                  70                  75                  80

Gly Val Thr Val Met Leu Phe Ser Met Val Arg Thr Val Glu Ser Cys
                85                  90                  95

Trp Tyr Phe Gly Ala Lys Phe Cys Thr Leu His Ser Cys Cys Asp Val
            100                 105                 110

Ala Phe Cys Tyr Ser Ser Val Leu His Leu Cys Phe Ile Cys Ile Asp
        115                 120                 125

Arg Tyr Ile Val Val Thr Asp Pro Leu Val Tyr Ala Thr Lys Phe Thr
    130                 135                 140

Val Ser Val Ser Gly Ile Cys Ile Ser Val Ser Trp Ile Leu Pro Leu
145                 150                 155                 160

Thr Tyr Ser Gly Ala Val Phe Tyr Thr Gly Val Asn Asp Asp Gly Leu

                165                 170                 175

Glu Glu Leu Val Ser Ala Leu Asn Cys Val Gly Gly Cys Gln Ile Ile
            180                 185                 190

Val Ser Gln Gly Trp Val Leu Ile Asp Phe Leu Leu Phe Phe Ile Pro
        195                 200                 205

Thr Leu Val Met Ile Ile Leu Tyr Ser Lys Ile Phe Leu Ile Ala Lys
    210                 215                 220

Gln Gln Ala Ile Lys Ile Glu Thr Thr Ser Ser Lys Val Glu Ser Ser
225                 230                 235                 240

Ser Glu Ser Tyr Lys Ile Arg Val Ala Lys Arg Glu Arg Lys Ala Ala
                245                 250                 255

Lys Thr Leu Gly Val Thr Val Leu Ala Phe Val Ile Ser Trp Leu Pro
            260                 265                 270

Tyr Thr Val Asp Ile Leu Ile Asp Ala Phe Met Gly Phe Leu Thr Pro
        275                 280                 285

Ala Tyr Ile Tyr Glu Ile Cys Cys Trp Ser Ala Tyr Tyr Asn Ser Ala
    290                 295                 300

Met Asn Pro Leu Ile Tyr Ala Leu Phe Tyr Pro Trp Phe Arg Lys Ala
305                 310                 315                 320

Ile Lys Leu Ile Leu Ser Gly Asp Val Leu Lys Ala Ser Ser Ser Thr
                325                 330                 335

Ile Ser Leu Phe Leu Glu
            340

<210> 19
<211> 1047
<212> DNA
<213> Homo sapiens

<220>
<221> huTAAR 9
<222> (1)..(1047)

<400> 19
atggtgaata atttctccca agctgaggct gtggagctgt gttacaagaa cgtgaacgaa
60

tcctgcatta aaactcctta ctcgccaggt cctcgatcta tcctctacgc cgtccttggt
120

tttggggctg tgctggcagc gtttggaaac ttactggtca tgattgctat ccttcacttc
180

aaacaactgc acacacctac aaactttctg attgcgtcgc tggcctgtgc tgacttcttg
240

gtgggagtca ctgtgatgcc cttcagcaca gtgaggtctg tggagagctg ttggtacttt
300

ggggacagtt actgtaaatt ccatacatgt tttgacacat ccttctgttt tgcttcttta
360

tttcatttat gctgtatctc tgttgataga tacattgctg ttactgatcc tctgacctat
420

ccaaccaagt ttactgtgtc agtttcaggg atatgcattg ttctttcctg gttcttttct
480

gtcacataca gcttttcgat cttttacacg ggagccaacg aagaaggaat tgaggaatta
540

gtagttgctc taacctgtgt aggaggctgc caggctccac tgaatcaaaa ctgggtccta
600

ctttgttttc ttctattctt tatacccaat gtcgccatgg tgtttatata cagtaagata
660

tttttggtgg ccaagcatca ggctaggaag atagaaagta cagccagcca agctcagtcc
720

tcctcagaga gttacaagga aagagtagca aaaagagaga gaaaggctgc caaaaccttg
780

ggaattgcta tggcagcatt tcttgtctct tggctaccat acctcgttga tgcagtgatt
840

gatgcttata tgaattttat aactcctcct tatgtttatg agattttagt ttggtgtgtt
900

tattataatt cagctatgaa ccccttgatt tatgctttct tttaccaatg gtttgggaag
960

gcaataaaac ttattgtaag cggcaaggtc ttaaggactg attcgtcaac aactaattta
1020

ttttctgaag aagtagagac agattaa
1047


<210>  20
<211>  348
<212>  PRT
<213>  Homo sapiens


<220>
<221>  huTAAR 9
<222>  (1)..(348)

<400>  20

Met Val Asn Asn Phe Ser Gln Ala Glu Ala Val Glu Leu Cys Tyr Lys
1               5                   10                  15


Asn Val Asn Glu Ser Cys Ile Lys Thr Pro Tyr Ser Pro Gly Pro Arg
            20                  25                  30


Ser Ile Leu Tyr Ala Val Leu Gly Phe Gly Ala Val Leu Ala Ala Phe
        35                  40                  45


Gly Asn Leu Leu Val Met Ile Ala Ile Leu His Phe Lys Gln Leu His
    50                  55                  60

Thr Pro Thr Asn Phe Leu Ile Ala Ser Leu Ala Cys Ala Asp Phe Leu
65                  70                  75                  80

Val Gly Val Thr Val Met Pro Phe Ser Thr Val Arg Ser Val Glu Ser
                85                  90                  95

Cys Trp Tyr Phe Gly Asp Ser Tyr Cys Lys Phe His Thr Cys Phe Asp
            100                 105                 110

Thr Ser Phe Cys Phe Ala Ser Leu Phe His Leu Cys Cys Ile Ser Val
        115                 120                 125

Asp Arg Tyr Ile Ala Val Thr Asp Pro Leu Thr Tyr Pro Thr Lys Phe
    130                 135                 140

Thr Val Ser Val Ser Gly Ile Cys Ile Val Leu Ser Trp Phe Phe Ser
145                 150                 155                 160

Val Thr Tyr Ser Phe Ser Ile Phe Tyr Thr Gly Ala Asn Glu Glu Gly
                165                 170                 175

Ile Glu Glu Leu Val Val Ala Leu Thr Cys Val Gly Gly Cys Gln Ala
            180                 185                 190

Pro Leu Asn Gln Asn Trp Val Leu Leu Cys Phe Leu Leu Phe Phe Ile
        195                 200                 205

Pro Asn Val Ala Met Val Phe Ile Tyr Ser Lys Ile Phe Leu Val Ala
    210                 215                 220

Lys His Gln Ala Arg Lys Ile Glu Ser Thr Ala Ser Gln Ala Gln Ser
225                 230                 235                 240

Ser Ser Glu Ser Tyr Lys Glu Arg Val Ala Lys Arg Glu Arg Lys Ala
                245                 250                 255

Ala Lys Thr Leu Gly Ile Ala Met Ala Ala Phe Leu Val Ser Trp Leu
            260                 265                 270

Pro Tyr Leu Val Asp Ala Val Ile Asp Ala Tyr Met Asn Phe Ile Thr
        275                 280                 285

Pro Pro Tyr Val Tyr Glu Ile Leu Val Trp Cys Val Tyr Tyr Asn Ser
    290                 295                 300

Ala Met Asn Pro Leu Ile Tyr Ala Phe Phe Tyr Gln Trp Phe Gly Lys
305                 310                 315                 320

Ala Ile Lys Leu Ile Val Ser Gly Lys Val Leu Arg Thr Asp Ser Ser
                325                 330                 335

Thr Thr Asn Leu Phe Ser Glu Glu Val Glu Thr Asp

            340                 345

<210> 21
<211> 18
<212> DNA
<213> Homo sapiens

<220>
<221> hTAAR 1_5_01
<222> (1)..(18)
<223> primer

<400> 21
atgatgccct tttgccac
18

<210> 22
<211> 29
<212> DNA
<213> Homo sapiens

<220>
<221> hTAAR 1_3_01
<222> (1)..(29)
<223> primer

<400> 22
ttatgaactc aattccaaaa ataatttac
29

<210> 23
<211> 22
<212> DNA
<213> Homo sapiens

<220>
<221> humGPR58_5_06
<222> (1)..(22)
<223> primer

<400> 23
catctacatc tggggtatct tg
22

<210> 24
<211> 21
<212> DNA
<213> Homo sapiens

<220>
<221> humGPR58_3_06
<222> (1)..(21)
<223> primer

<400> 24
tgcttcaatt tattcatgca g
21

<210> 25
<211> 24
<212> DNA

<213>   Homo sapiens


<220>
<221>   humGPR57_5_01
<222>   (1)..(24)
<223>   primer

<400>   25
atggatctaa cttatattcc cgaa
24


<210>   26
<211>   22
<212>   DNA
<213>   Homo sapiens


<220>
<221>   humGPR57_3_01
<222>   (1)..(22)
<223>   primer

<400>   26
ttaatgtgct tcaggaaaca aa
22


<210>   27
<211>   19
<212>   DNA
<213>   Homo sapiens


<220>
<221>   hTAAR 4_5_01
<222>   (1)..(19)
<223>   primer

<400>   27
atgatgaatt tgcctgacc
19


<210>   28
<211>   19
<212>   DNA
<213>   Homo sapiens


<220>
<221>   hTAAR 4_3_01
<222>   (1)..(19)
<223>   primer

<400>   28
ctaagcatgg gcagaaaac
19


<210>   29
<211>   20
<212>   DNA
<213>   Homo sapiens


<220>
<221>   humPNR_5_01
<222>   (1)..(20)

<223>  primer

<400>  29
atgagagctg tcttcatcca
20

<210>  30
<211>  23
<212>  DNA
<213>  Homo sapiens

<220>
<221>  humPNR_3_01
<222>  (1)..(23)
<223>  primer

<400>  30
tcattcttgg tacaaatcaa cag
23

<210>  31
<211>  19
<212>  DNA
<213>  Homo sapiens

<220>
<221>  hTAAR 6_5_01
<222>  (1)..(19)
<223>  primer

<400>  31
atgagcagca attcatccc
19

<210>  32
<211>  28
<212>  DNA
<213>  Homo sapiens

<220>
<221>  hTAAR 6_3_01
<222>  (1)..(28)
<223>  primer

<400>  32
ttatatatgt tcagaaaaca aattcatg
28

<210>  33
<211>  19
<212>  DNA
<213>  Homo sapiens

<220>
<221>  hTAAR 8_5_01
<222>  (1)..(19)
<223>  primer

<400>  33
atgaccagca atttttccc
19

<210>  34
<211>  29
<212>  DNA
<213>  Homo sapiens

<220>
<221>  hTAAR 8_3_01
<222>  (1)..(29)
<223>  primer

<400>  34
ttattctaaa aataaactaa tggttgatg
29

<210>  35
<211>  22
<212>  DNA
<213>  Homo sapiens

<220>
<221>  hTAAR 9_5_01
<222>  (1)..(22)
<223>  primer

<400>  35
atggtgaata atttctccca ag
22

<210>  36
<211>  28
<212>  DNA
<213>  Homo sapiens

<220>
<221>  hTAAR 9_3_01
<222>  (1)..(28)
<223>  primer

<400>  36
ttaatctgtc tctacttctt cagaaaat
28

### Annex to the application documents - subsequently filed sequences listing

SEQUENCE LISTING

<110>  F. Hoffmann-La Roche

<120>  Human Trace Amine Associated Receptors

<130>  22645

<140>  EP 05013871.8
<141>  2005-06-28

<160>  36

<170>  PatentIn version 3.3

<210>  1
<211>  16
<212>  PRT
<213>  Homo sapiens


<220>
<221>  VARIANT
<222>  (3)..(4)
<223>  Xaa may be any naturally occuring amino acid

<220>
<221>  VARIANT
<222>  (7)..(8)
<223>  Xaa may be any naturally occuring amino acid

<220>
<221>  VARIANT
<222>  (10)..(12)
<223>  Xaa may be any naturally occuring amino acid

<220>
<221>  VARIANT
<222>  (14)..(14)
<223>  Xaa may be any naturally occuring amino acid

<400>  1

Asn Ser Xaa Xaa Asn Pro Xaa Xaa Tyr Xaa Xaa Xaa Tyr Xaa Trp Phe
1               5                   10                  15


<210>  2
<211>  1020
<212>  DNA
<213>  Homo sapiens

<400>  2
atgatgccct tttgccacaa tataattaat atttcctgtg tgaaaaacaa ctggtcaaat      60

gatgtccgtg cttccctgta cagtttaatg gtgctcataa ttctgaccac actcgttggc     120

aatctgatag ttattgtttc tatatcacac ttcaaacaac ttcatacccc aacaaattgg     180

ctcattcatt ccatggccac tgtggacttt cttctggggt gtctggtcat gccttacagt     240

atggtgagat ctgctgagca ctgttggtat tttggagaag tcttctgtaa aattcacaca     300

agcaccgaca ttatgctgag ctcagcctcc attttccatt tgtctttcat ctccattgac     360

cgctactatg ctgtgtgtga tccactgaga tataaagcca agatgaatat cttggttatt     420

tgtgtgatga tcttcattag ttggagtgtc cctgctgttt ttgcatttgg aatgatcttt 480
ctggagctaa acttcaaagg cgctgaagag atatattaca aacatgttca ctgcagagga 540
ggttgctctg tcttctttag caaaatatct ggggtactga cctttatgac ttctttttat 600
atacctggat ctattatgtt atgtgtctat tacagaatat atcttatcgc taaagaacag 660
gcaagattaa ttagtgatgc caatcagaag ctccaaattg gattggaaat gaaaaatgga 720
atttcacaaa gcaaagaaag gaaagctgtg aagacattgg ggattgtgat gggagttttc 780
ctaatatgct ggtgcccttt ctttatctgt acagtcatgg acccttttct tcactacatt 840
attccaccta ctttgaatga tgtattgatt tggtttggct acttgaactc tacatttaat 900
ccaatggttt atgcattttt ctatccttgg tttagaaaag cactgaagat gatgctgttt 960
ggtaaaattt tccaaaaaga ttcatccagg tgtaaattat ttttggaatt gagttcataa 1020

<210> 3
<211> 339
<212> PRT
<213> Homo sapiens

<400> 3

Met Met Pro Phe Cys His Asn Ile Ile Asn Ile Ser Cys Val Lys Asn
1               5                   10                  15

Asn Trp Ser Asn Asp Val Arg Ala Ser Leu Tyr Ser Leu Met Val Leu
            20                  25                  30

Ile Ile Leu Thr Thr Leu Val Gly Asn Leu Ile Val Ile Val Ser Ile
        35                  40                  45

Ser His Phe Lys Gln Leu His Thr Pro Thr Asn Trp Leu Ile His Ser
    50                  55                  60

Met Ala Thr Val Asp Phe Leu Leu Gly Cys Leu Val Met Pro Tyr Ser
65                  70                  75                  80

Met Val Arg Ser Ala Glu His Cys Trp Tyr Phe Gly Glu Val Phe Cys
                85                  90                  95

Lys Ile His Thr Ser Thr Asp Ile Met Leu Ser Ser Ala Ser Ile Phe
            100                 105                 110

His Leu Ser Phe Ile Ser Ile Asp Arg Tyr Tyr Ala Val Cys Asp Pro
        115                 120                 125

Leu Arg Tyr Lys Ala Lys Met Asn Ile Leu Val Ile Cys Val Met Ile
    130                 135                 140

Phe Ile Ser Trp Ser Val Pro Ala Val Phe Ala Phe Gly Met Ile Phe

145                 150                 155                 160

Leu Glu Leu Asn Phe Lys Gly Ala Glu Glu Ile Tyr Tyr Lys His Val
                165                 170                 175

His Cys Arg Gly Gly Cys Ser Val Phe Phe Ser Lys Ile Ser Gly Val
            180                 185                 190

Leu Thr Phe Met Thr Ser Phe Tyr Ile Pro Gly Ser Ile Met Leu Cys
        195                 200                 205

Val Tyr Tyr Arg Ile Tyr Leu Ile Ala Lys Glu Gln Ala Arg Leu Ile
    210                 215                 220

Ser Asp Ala Asn Gln Lys Leu Gln Ile Gly Leu Glu Met Lys Asn Gly
225                 230                 235                 240

Ile Ser Gln Ser Lys Glu Arg Lys Ala Val Lys Thr Leu Gly Ile Val
                245                 250                 255

Met Gly Val Phe Leu Ile Cys Trp Cys Pro Phe Phe Ile Cys Thr Val
            260                 265                 270

Met Asp Pro Phe Leu His Tyr Ile Ile Pro Pro Thr Leu Asn Asp Val
        275                 280                 285

Leu Ile Trp Phe Gly Tyr Leu Asn Ser Thr Phe Asn Pro Met Val Tyr
    290                 295                 300

Ala Phe Phe Tyr Pro Trp Phe Arg Lys Ala Leu Lys Met Met Leu Phe
305                 310                 315                 320

Gly Lys Ile Phe Gln Lys Asp Ser Ser Arg Cys Lys Leu Phe Leu Glu
                325                 330                 335

Leu Ser Ser

<210>  4
<211>  1056
<212>  DNA
<213>  Homo sapiens

<400>  4
atggctgtct catcagagca acatgaactt tcacatttca aaagaacaca gacaaaaaag      60

gaaaaattca attgctctga atatggaaat agatcttgcc cagaaaatga aagatctctg     120

ggtgtccgag tggctatgta ttcatttatg gcaggatcca tattcatcac aatatttggc     180

aatcttgcca tgataatttc catttcctac ttcaagcagc ttcacacacc aaccaacttc     240

ctcatcctct ccatggccat cactgatttc ctcctgggat tcaccatcat gccatatagt     300

atgatcagat cggtggagaa ctgctggtat tttgggctta cattttgcaa gatttattat 360

agttttgacc tgatgcttag cataacatcc atttttcatc tttgctcagt ggccattgat 420

agattttatg ctatatgtta cccattactt tattccacca aaataactat tccagtcatt 480

aaaagattgc tacttctatg ttggtcggtc cctggagcat ttgccttcgg ggtggtcttc 540

tcagaggcct atgcagatgg aatagagggc tatgacatct tggttgcttg ttccagttcc 600

tgcccagtga tgttcaacaa gctatggggg accaccttgt ttatggcagg tttcttcact 660

cctgggtcta tgatggtggg gatttatggc aaaatttttg cagtatccag aaaacatgct 720

catgccatca ataacttgcg agaaaatcaa aataatcaag tgaagaaaga caaaaaagct 780

gccaaaactt taggaatagt gataggagtt ttcttattat gttggtttcc ttgtttcttc 840

acaattttat tggatccctt tttgaacttc tctactcctg tagttttgtt tgatgccttg 900

acatggtttg gctattttaa ctccacatgt aatccgttaa tatatggttt cttctatccc 960

tggtttcgca gagcactgaa gtacattttg ctaggtaaaa ttttcagctc atgtttccat 1020

aatactattt tgtgtatgca aaaagaaagt gagtag 1056

<210> 5
<211> 351
<212> PRT
<213> Homo sapiens

<400> 5

Met Ala Val Ser Ser Glu Gln His Glu Leu Ser His Phe Lys Arg Thr
1               5                   10                  15

Gln Thr Lys Lys Glu Lys Phe Asn Cys Ser Glu Tyr Gly Asn Arg Ser
            20                  25                  30

Cys Pro Glu Asn Glu Arg Ser Leu Gly Val Arg Val Ala Met Tyr Ser
        35                  40                  45

Phe Met Ala Gly Ser Ile Phe Ile Thr Ile Phe Gly Asn Leu Ala Met
    50                  55                  60

Ile Ile Ser Ile Ser Tyr Phe Lys Gln Leu His Thr Pro Thr Asn Phe
65                  70                  75                  80

Leu Ile Leu Ser Met Ala Ile Thr Asp Phe Leu Leu Gly Phe Thr Ile
                85                  90                  95

Met Pro Tyr Ser Met Ile Arg Ser Val Glu Asn Cys Trp Tyr Phe Gly
            100                 105                 110

Leu Thr Phe Cys Lys Ile Tyr Tyr Ser Phe Asp Leu Met Leu Ser Ile
        115                 120                 125

Thr Ser Ile Phe His Leu Cys Ser Val Ala Ile Asp Arg Phe Tyr Ala
    130                 135                 140

Ile Cys Tyr Pro Leu Leu Tyr Ser Thr Lys Ile Thr Ile Pro Val Ile
145                 150                 155                 160

Lys Arg Leu Leu Leu Leu Cys Trp Ser Val Pro Gly Ala Phe Ala Phe
                165                 170                 175

Gly Val Val Phe Ser Glu Ala Tyr Ala Asp Gly Ile Glu Gly Tyr Asp
            180                 185                 190

Ile Leu Val Ala Cys Ser Ser Ser Cys Pro Val Met Phe Asn Lys Leu
        195                 200                 205

Trp Gly Thr Thr Leu Phe Met Ala Gly Phe Phe Thr Pro Gly Ser Met
    210                 215                 220

Met Val Gly Ile Tyr Gly Lys Ile Phe Ala Val Ser Arg Lys His Ala
225                 230                 235                 240

His Ala Ile Asn Asn Leu Arg Glu Asn Gln Asn Asn Gln Val Lys Lys
                245                 250                 255

Asp Lys Lys Ala Ala Lys Thr Leu Gly Ile Val Ile Gly Val Phe Leu
            260                 265                 270

Leu Cys Trp Phe Pro Cys Phe Phe Thr Ile Leu Leu Asp Pro Phe Leu
        275                 280                 285

Asn Phe Ser Thr Pro Val Val Leu Phe Asp Ala Leu Thr Trp Phe Gly
    290                 295                 300

Tyr Phe Asn Ser Thr Cys Asn Pro Leu Ile Tyr Gly Phe Phe Tyr Pro
305                 310                 315                 320

Trp Phe Arg Arg Ala Leu Lys Tyr Ile Leu Leu Gly Lys Ile Phe Ser
                325                 330                 335

Ser Cys Phe His Asn Thr Ile Leu Cys Met Gln Lys Glu Ser Glu
            340                 345                 350

<210>  6
<211>  1030
<212>  DNA
<213>  Homo sapiens

<400>  6
atggatctaa cttatattcc cgaagaccta tccagttgtc caaaatttgt aaataaatcc      60

tgtcctccca ccaaccgctc ttttcatgtc caggtgataa tgtattcggt tatgactgga     120

gccatgatta tcactattcg gaaacttggt tataatggtt tccatatcgc atttcaaaca 180

gcttcactct cccacaaact ttctgatcct ctccatggca accacggact ttctgctggg 240

ttttgtcatt atgccataca gcataatgcg atcagtggag agttgctggt actttgggga 300

tggcttttgt aaattccaca caagctttga catgatgctc agactgacct ccattttcca 360

cctctgttcc attgctattg accgatttta tgccgtgtgt taccctttac attacacaac 420

caaaatgacg aactccacca taaagcaact gctggcattt tgctggtcag ttcctgctct 480

tttttctttt ggtttagttc tatctgaggc cgatgtttcc ggtatgcaga gctataagat 540

acttgttgct tgcttcaatt tctgtgccct tactttcaac aaattctggg ggacaatatt 600

gttcactaca tgtttcttta cccctggctc catcatggtt ggtatttatg gcaaaatctt 660

tatcgtttcc aaacagcatg ctcgagtcat cagccatgtg cctgaaaaca caaagggggc 720

agtgaaaaaa cacctatcca agaaaaagga caggaaagca gcgaagacac tgggtatagt 780

aatgggggtg tttctggctt gctggttgcc ttgttttctt gctgttctga ttgacccata 840

cctagactac tccactccca tactaatatt ggatctttta gtgtggctcc ggtacttcaa 900

ctctacttgc aaccctctta ttcatggctt ttttaatcca tggtttcaga aagcattcaa 960

gtacatagtg tcaggaaaaa tatttagctc ccattcagaa actgcaaatt tgtttcctga 1020

agcacattaa 1030

<210> 7
<211> 1032
<212> DNA
<213> Homo sapiens

<400> 7
atggatctaa cttatattcc cgaagaccta tccagttgtc caaaatttgt aaataaatcc 60

tgtcctccca ccaaccgctc ttttcatgtc caggtgataa tgtattcggt tatgactgga 120

gccatgatta tcaccatatt cggaaacttg gttataatgg tttccatatc gcatttcaaa 180

cagcttcact ctcccacaaa ctttctgatc ctctccatgg caaccacgga ctttctgctg 240

ggttttgtca ttatgccata cagcataatg cgatcagtgg agagttgctg gtactttggg 300

gatggctttt gtaaattcca cacaagcttt gacatgatgc tcagactgac ctccattttc 360

cacctctgtt ccattgctat tgaccgattt tatgccgtgt gttacccttt acattacaca 420

accaaaatga cgaactccac cataaagcaa ctgctggcat tttgctggtc agttcctgct 480

cttttttctt ttggtttagt tctatctgag gccgatgttt ccggtatgca gagctataag 540

atacttgttg cttgcttcaa tttctgtgcc cttactttca acaaattctg ggggacaata 600

ttgttcacta catgtttctt tacccctggc tccatcatgg ttggtattta tggcaaaatc 660

tttatcgttt ccaaacagca tgctcgagtc atcagccatg tgcctgaaaa cacaaagggg 720

gcagtgaaaa aacacctatc caagaaaaag gacaggaaag cagcgaagac actgggtata 780

gtaatggggg tgtttctggc ttgctggttg ccttgttttc ttgctgttct gattgaccca     840

tacctagact actccactcc catactaata ttggatcttt tagtgtggct ccggtacttc     900

aactctactt gcaaccctct tattcatggc ttttttaatc catggtttca gaaagcattc     960

aagtacatag tgtcaggaaa aatatttagc tcccattcag aaactgcaaa tttgtttcct    1020

gaagcacatt aa                                                        1032

<210>  8
<211>  343
<212>  PRT
<213>  Homo sapiens

<400>  8

Met Asp Leu Thr Tyr Ile Pro Glu Asp Leu Ser Ser Cys Pro Lys Phe
1               5                   10                  15

Val Asn Lys Ser Cys Pro Pro Thr Asn Arg Ser Phe His Val Gln Val
            20                  25                  30

Ile Met Tyr Ser Val Met Thr Gly Ala Met Ile Ile Thr Ile Phe Gly
        35                  40                  45

Asn Leu Val Ile Met Val Ser Ile Ser His Phe Lys Gln Leu His Ser
    50                  55                  60

Pro Thr Asn Phe Leu Ile Leu Ser Met Ala Thr Thr Asp Phe Leu Leu
65                  70                  75                  80

Gly Phe Val Ile Met Pro Tyr Ser Ile Met Arg Ser Val Glu Ser Cys
                85                  90                  95

Trp Tyr Phe Gly Asp Gly Phe Cys Lys Phe His Thr Ser Phe Asp Met
            100                 105                 110

Met Leu Arg Leu Thr Ser Ile Phe His Leu Cys Ser Ile Ala Ile Asp
        115                 120                 125

Arg Phe Tyr Ala Val Cys Tyr Pro Leu His Tyr Thr Thr Lys Met Thr
    130                 135                 140

Asn Ser Thr Ile Lys Gln Leu Leu Ala Phe Cys Trp Ser Val Pro Ala
145                 150                 155                 160

Leu Phe Ser Phe Gly Leu Val Leu Ser Glu Ala Asp Val Ser Gly Met
                165                 170                 175

Gln Ser Tyr Lys Ile Leu Val Ala Cys Phe Asn Phe Cys Ala Leu Thr
            180                 185                 190

Phe Asn Lys Phe Trp Gly Thr Ile Leu Phe Thr Thr Cys Phe Phe Thr
        195                 200                 205

Pro Gly Ser Ile Met Val Gly Ile Tyr Gly Lys Ile Phe Ile Val Ser
    210                 215                 220

Lys Gln His Ala Arg Val Ile Ser His Val Pro Glu Asn Thr Lys Gly
225                 230                 235                 240

Ala Val Lys Lys His Leu Ser Lys Lys Lys Asp Arg Lys Ala Ala Lys
                245                 250                 255

Thr Leu Gly Ile Val Met Gly Val Phe Leu Ala Cys Trp Leu Pro Cys
            260                 265                 270

Phe Leu Ala Val Leu Ile Asp Pro Tyr Leu Asp Tyr Ser Thr Pro Ile
        275                 280                 285

Leu Ile Leu Asp Leu Leu Val Trp Leu Arg Tyr Phe Asn Ser Thr Cys
    290                 295                 300

Asn Pro Leu Ile His Gly Phe Phe Asn Pro Trp Phe Gln Lys Ala Phe
305                 310                 315                 320

Lys Tyr Ile Val Ser Gly Lys Ile Phe Ser Ser His Ser Glu Thr Ala
                325                 330                 335

Asn Leu Phe Pro Glu Ala His
            340

<210>  9
<211>  1049
<212>  DNA
<213>  Homo sapiens

<400>  9
atgatgaatt tgcctgaccc tcagaacccc ccaacagtac aattttgctt tagttcagtt      60

aacaattcat gccctagaaa tgtgaggccg gtgctgagtg tctgggccat gtacctggtc     120

atgatcgggt ctatagtgat gacaatgctg ggcaacatga tcgtaatgat ttccatcgct     180

cacttcaagc agctccactc cccgaccaac ttcttgatcc tctccatggc catcactgac     240

tttttgctga gctgtgtggt catgcccttc agtgtgatca catccatcga gtcctgctgg     300

tattttggag acctcttttg caaagtccac agctgctgtg acatcatact ctgcaccacc     360

tccatttttc acctctgctt catctcagtt gaccgttacg atgctgtttg agacccattg     420

caatatgtca ccagaattac catccctgtc atagaactct ttctactcat cagttggtcc     480

attcccatcc tttttgcctt tggcctggta ttctcaaaac taaacataat tggtgcagaa     540

gagtttgttg cagccattga ttgcacaggt ttgtgtgtgt taatatttaa taagctctgg     600

gggggtactg gcctccttta tagctttctt tctcccaggg acaaccacgg tgggaattta 660

catacatatt tttacagtag ccaggaagca tgccatgcaa attggcacag gttctaggac 720

taaacaggct gggtcagaaa gcaaaaaaaa aggcatcctc taaaacagaa agcaaggcca 780

ccaggacctt aggcatagtc atgggagtgt ttgtgttgtg ctggctgccc ttctttgtct 840

tgacgatcac agatcctttc attaatttta caacccttga agatctgtac aatgtcttcc 900

tctggctagg ctatttcaac tctgctttca atcccatttt atatggcatg ctttatcctt 960

ggtttcgcaa ggcattgagg atgattgtca caggcatgat cttccaccct gactcttcca 1020

ccctaagcct gttttctgcc catgcttag 1049

<210> 10
<211> 1047
<212> DNA
<213> Homo sapiens

<400> 10
atgatgaatt tgcctgaccc tcagaacccc ccaacagtac aattttgctt tagttcagtt 60

aacaattcat gccctagaaa tgtgaggccg gtgctgagtg tctgggccat gtacctggtc 120

atgatcgggt ctatagtgat gacaatgctg ggcaacatga tcgtaatgat ttccatcgct 180

cacttcaagc agctccactc cccgaccaac ttcttgatcc tctccatggc catcactgac 240

tttttgctga gctgtgtggt catgcccttc agtgtgatca catccatcga gtcctgctgg 300

tattttggag acctcttttg caaagtccac agctgctgtg acatcatact ctgcaccacc 360

tccatttttc acctctgctt catctcagtt gaccgttacg atgctgtttg tgacccattg 420

caatatgtca ccagaattac catccctgtc atagaactct ttctactcat cagttggtcc 480

attcccatcc tttttgcctt tggcctggta ttctcaaaac taaacataat tggtgcagaa 540

gagtttgttg cagccattga ttgcacaggt ttgtgtgtgt taatatttaa taagctctgg 600

ggggtactgg cctcctttat agctttcttt ctcccaggga caaccacggt gggaatttac 660

atacatattt ttacagtagc caggaagcat gccatgcaaa ttggcacagg ttctaggact 720

aaacaggctg ggtcagaaag caaaaaaaag gcatcctcta aaacagaaag caaggccacc 780

aggaccttag gcatagtcat gggagtgttt gtgttgtgct ggctgccctt ctttgtcttg 840

acgatcacag atcctttcat taattttaca acccttgaag atctgtacaa tgtcttcctc 900

tggctaggct atttcaactc tgctttcaat cccattttat atggcatgct ttatccttgg 960

tttcgcaagg cattgaggat gattgtcaca ggcatgatct tccaccctga ctcttccacc 1020

ctaagcctgt tttctgccca tgcttag 1047

<210> 11
<211> 348
<212> PRT
<213> Homo sapiens

<400> 11

Met Met Asn Leu Pro Asp Pro Gln Asn Pro Pro Thr Val Gln Phe Cys
1               5                   10                  15

Phe Ser Ser Val Asn Asn Ser Cys Pro Arg Asn Val Arg Pro Val Leu
            20                  25                  30

Ser Val Trp Ala Met Tyr Leu Val Met Ile Gly Ser Ile Val Met Thr
        35                  40                  45

Met Leu Gly Asn Met Ile Val Met Ile Ser Ile Ala His Phe Lys Gln
    50                  55                  60

Leu His Ser Pro Thr Asn Phe Leu Ile Leu Ser Met Ala Ile Thr Asp
65                  70                  75                  80

Phe Leu Leu Ser Cys Val Val Met Pro Phe Ser Val Ile Thr Ser Ile
                85                  90                  95

Glu Ser Cys Trp Tyr Phe Gly Asp Leu Phe Cys Lys Val His Ser Cys
            100                 105                 110

Cys Asp Ile Ile Leu Cys Thr Thr Ser Ile Phe His Leu Cys Phe Ile
        115                 120                 125

Ser Val Asp Arg Tyr Asp Ala Val Cys Asp Pro Leu Gln Tyr Val Thr
    130                 135                 140

Arg Ile Thr Ile Pro Val Ile Glu Leu Phe Leu Leu Ile Ser Trp Ser
145                 150                 155                 160

Ile Pro Ile Leu Phe Ala Phe Gly Leu Val Phe Ser Lys Leu Asn Ile
                165                 170                 175

Ile Gly Ala Glu Glu Phe Val Ala Ala Ile Asp Cys Thr Gly Leu Cys
            180                 185                 190

Val Leu Ile Phe Asn Lys Leu Trp Gly Val Leu Ala Ser Phe Ile Ala
        195                 200                 205

Phe Phe Leu Pro Gly Thr Thr Thr Val Gly Ile Tyr Ile His Ile Phe
    210                 215                 220

Thr Val Ala Arg Lys His Ala Met Gln Ile Gly Thr Gly Ser Arg Thr
225                 230                 235                 240

Lys Gln Ala Gly Ser Glu Ser Lys Lys Lys Ala Ser Ser Lys Thr Glu
                245                 250                 255

Ser Lys Ala Thr Arg Thr Leu Gly Ile Val Met Gly Val Phe Val Leu

260                 265                 270

Cys Trp Leu Pro Phe Phe Val Leu Thr Ile Thr Asp Pro Phe Ile Asn
        275                 280                 285

Phe Thr Thr Leu Glu Asp Leu Tyr Asn Val Phe Leu Trp Leu Gly Tyr
    290                 295                 300

Phe Asn Ser Ala Phe Asn Pro Ile Leu Tyr Gly Met Leu Tyr Pro Trp
305                 310                 315                 320

Phe Arg Lys Ala Leu Arg Met Ile Val Thr Gly Met Ile Phe His Pro
                325                 330                 335

Asp Ser Ser Thr Leu Ser Leu Phe Ser Ala His Ala
            340                 345

<210>  12
<211>  1014
<212>  DNA
<213>  Homo sapiens

<400>  12
   atgagagctg tcttcatcca aggtgctgaa gagcaccctg cggcattctg ctaccaggtg      60
   aatgggtctt gccccaggac agtacatact ctgggcatcc agttggtcat ctacctggcc     120
   tgtgcagcag gcatgctgat tatcgtgcta gggaatgtat ttgtggcatt tgctgtgtcc     180
   tacttcaaag cgcttcacac gcccaccaac ttcctgctgc tctccctggc cctggctgac     240
   atgtttctgg gtctgctggt gctgcccctc agcaccattc gctcagtgga gagctgctgg     300
   ttcttcgggg acttcctctg ccgcctgcac acctacctgg acaccctctt ctgcctcacc     360
   tccatcttcc atctctgttt catttccatt gaccgccact gtgccatctg tgaccccctg     420
   ctctatccct ccaagttcac agtgagggtg gctctcaggt acatcctggc aggatggggg     480
   gtgcccgcag catacacttc gttattcctc tacacagatg tggtagagac aaggctcagc     540
   cagtggctgg aagagatgcc ttgtgtgggc agttgccagc tgctgctcaa taaattttgg     600
   ggctggttaa acttcccttt gttctttgtc ccctgcctca ttatgatcag cttgtatgtg     660
   aagatctttg tggttgctac cagacaggct cagcagatta ccacattgag caaaagcctg     720
   gctggggctg ccaagcatga gagaaaagct gccaagaccc tgggcattgc tgtgggcata     780
   tacctcttgt gctggctgcc cttcaccata gacacgatgg tcgacagcct ccttcacttt     840
   atcacacccc cactggtctt tgacatcttt atctggtttg cttacttcaa ctcagcctgc     900
   aaccccatca tctatgtctt ttcctaccag tggtttcgga aggcactgaa actcacactg     960
   agccagaagg tcttctcacc gcagacacgc actgttgatt tgtaccaaga atga          1014

<210>  13
<211>  337

<212>  PRT
<213>  Homo sapiens

<400>  13

Met Arg Ala Val Phe Ile Gln Gly Ala Glu Glu His Pro Ala Ala Phe
1               5                   10                  15

Cys Tyr Gln Val Asn Gly Ser Cys Pro Arg Thr Val His Thr Leu Gly
            20                  25                  30

Ile Gln Leu Val Ile Tyr Leu Ala Cys Ala Ala Gly Met Leu Ile Ile
        35                  40                  45

Val Leu Gly Asn Val Phe Val Ala Phe Ala Val Ser Tyr Phe Lys Ala
    50                  55                  60

Leu His Thr Pro Thr Asn Phe Leu Leu Leu Ser Leu Ala Leu Ala Asp
65                  70                  75                  80

Met Phe Leu Gly Leu Leu Val Leu Pro Leu Ser Thr Ile Arg Ser Val
                85                  90                  95

Glu Ser Cys Trp Phe Phe Gly Asp Phe Leu Cys Arg Leu His Thr Tyr
            100                 105                 110

Leu Asp Thr Leu Phe Cys Leu Thr Ser Ile Phe His Leu Cys Phe Ile
        115                 120                 125

Ser Ile Asp Arg His Cys Ala Ile Cys Asp Pro Leu Leu Tyr Pro Ser
    130                 135                 140

Lys Phe Thr Val Arg Val Ala Leu Arg Tyr Ile Leu Ala Gly Trp Gly
145                 150                 155                 160

Val Pro Ala Ala Tyr Thr Ser Leu Phe Leu Tyr Thr Asp Val Val Glu
                165                 170                 175

Thr Arg Leu Ser Gln Trp Leu Glu Glu Met Pro Cys Val Gly Ser Cys
            180                 185                 190

Gln Leu Leu Leu Asn Lys Phe Trp Gly Trp Leu Asn Phe Pro Leu Phe
        195                 200                 205

Phe Val Pro Cys Leu Ile Met Ile Ser Leu Tyr Val Lys Ile Phe Val
    210                 215                 220

Val Ala Thr Arg Gln Ala Gln Gln Ile Thr Thr Leu Ser Lys Ser Leu
225                 230                 235                 240

Ala Gly Ala Ala Lys His Glu Arg Lys Ala Ala Lys Thr Leu Gly Ile

245                 250                 255

Ala Val Gly Ile Tyr Leu Leu Cys Trp Leu Pro Phe Thr Ile Asp Thr
            260                 265                 270

Met Val Asp Ser Leu Leu His Phe Ile Thr Pro Pro Leu Val Phe Asp
        275                 280                 285

Ile Phe Ile Trp Phe Ala Tyr Phe Asn Ser Ala Cys Asn Pro Ile Ile
    290                 295                 300

Tyr Val Phe Ser Tyr Gln Trp Phe Arg Lys Ala Leu Lys Leu Thr Leu
305                 310                 315                 320

Ser Gln Lys Val Phe Ser Pro Gln Thr Arg Thr Val Asp Leu Tyr Gln
                325                 330                 335

Glu

<210>  14
<211>  1038
<212>  DNA
<213>  Homo sapiens

<400>  14
atgagcagca attcatccct gctggtggct gtgcagctgt gctacgcgaa cgtgaatggg      60
tcctgtgtga aaatcccctt ctcgccggga tcccgggtga ttctgtacat agtgtttggc     120
tttggggctg tgctggctgt gtttggaaac ctcctggtga tgatttcaat cctccatttc     180
aagcagctgc actctccgac caattttctc gttgcctctc tggcctgcgc tgatttcttg     240
gtgggtgtga ctgtgatgcc cttcagcatg gtcaggacgg tggagagctg ctggtatttt     300
gggaggagtt tttgtacttt ccacacctgc tgtgatgtgg cattttgtta ctcttctctc     360
tttcacttgt gcttcatctc catcgacagg tacattgcgg ttactgaccc cctggtctat     420
cctaccaagt tcaccgtatc tgtgtcagga atttgcatca gcgtgtcctg gatcctgccc     480
ctcatgtaca gcggtgctgt gttctacaca ggtgtctatg acgatgggct ggaggaatta     540
tctgatgccc taaactgtat aggaggttgt cagaccgttg taaatcaaaa ctgggtgttg     600
acagattttc tatccttctt tatacctacc tttattatga taattctgta tggtaacata     660
tttcttgtgg ctagacgaca ggcgaaaaag atagaaaata ctggtagcaa gacagaatca     720
tcctcagaga gttacaaagc cagagtggcc aggagagaga gaaaagcagc taaaaccctg     780
ggggtcacag tggtagcatt tatgatttca tggttaccat atagcattga ttcattaatt     840
gatgccttta tgggctttat aacccctgcc tgtatttatg agatttgctg ttggtgtgct     900
tattataact cagccatgaa tcctttgatt tatgctttat tttacccatg gtttaggaaa     960
gcaataaaag ttattgtaac tggtcaggtt ttaaagaaca gttcagcaac catgaatttg    1020

ttttctgaac atatataa                                                    1038

<210>  15
<211>  345
<212>  PRT
<213>  Homo sapiens

<400>  15

Met Ser Ser Asn Ser Ser Leu Leu Val Ala Val Gln Leu Cys Tyr Ala
1               5                   10                  15

Asn Val Asn Gly Ser Cys Val Lys Ile Pro Phe Ser Pro Gly Ser Arg
            20                  25                  30

Val Ile Leu Tyr Ile Val Phe Gly Phe Gly Ala Val Leu Ala Val Phe
        35                  40                  45

Gly Asn Leu Leu Val Met Ile Ser Ile Leu His Phe Lys Gln Leu His
    50                  55                  60

Ser Pro Thr Asn Phe Leu Val Ala Ser Leu Ala Cys Ala Asp Phe Leu
65                  70                  75                  80

Val Gly Val Thr Val Met Pro Phe Ser Met Val Arg Thr Val Glu Ser
                85                  90                  95

Cys Trp Tyr Phe Gly Arg Ser Phe Cys Thr Phe His Thr Cys Cys Asp
            100                 105                 110

Val Ala Phe Cys Tyr Ser Ser Leu Phe His Leu Cys Phe Ile Ser Ile
        115                 120                 125

Asp Arg Tyr Ile Ala Val Thr Asp Pro Leu Val Tyr Pro Thr Lys Phe
    130                 135                 140

Thr Val Ser Val Ser Gly Ile Cys Ile Ser Val Ser Trp Ile Leu Pro
145                 150                 155                 160

Leu Met Tyr Ser Gly Ala Val Phe Tyr Thr Gly Val Tyr Asp Asp Gly
                165                 170                 175

Leu Glu Glu Leu Ser Asp Ala Leu Asn Cys Ile Gly Gly Cys Gln Thr
            180                 185                 190

Val Val Asn Gln Asn Trp Val Leu Thr Asp Phe Leu Ser Phe Phe Ile
        195                 200                 205

Pro Thr Phe Ile Met Ile Ile Leu Tyr Gly Asn Ile Phe Leu Val Ala
    210                 215                 220

Arg Arg Gln Ala Lys Lys Ile Glu Asn Thr Gly Ser Lys Thr Glu Ser
225                 230                 235                 240

Ser Ser Glu Ser Tyr Lys Ala Arg Val Ala Arg Arg Glu Arg Lys Ala
                245                 250                 255

Ala Lys Thr Leu Gly Val Thr Val Val Ala Phe Met Ile Ser Trp Leu
            260                 265                 270

Pro Tyr Ser Ile Asp Ser Leu Ile Asp Ala Phe Met Gly Phe Ile Thr
        275                 280                 285

Pro Ala Cys Ile Tyr Glu Ile Cys Cys Trp Cys Ala Tyr Tyr Asn Ser
    290                 295                 300

Ala Met Asn Pro Leu Ile Tyr Ala Leu Phe Tyr Pro Trp Phe Arg Lys
305                 310                 315                 320

Ala Ile Lys Val Ile Val Thr Gly Gln Val Leu Lys Asn Ser Ser Ala
                325                 330                 335

Thr Met Asn Leu Phe Ser Glu His Ile
            340                 345

<210>  16
<211>  210
<212>  DNA
<213>  Homo sapiens

<400>  16
   gtggtagcat tttaaacctc atggttacta tatatgatga ttgattcatt cgttggttct      60
   ctttggggtt ttatcatacc ttcctatgtt tatgagatat tttcctagtt tggttactct     120
   aactgtgttc caaatacttt gatttacgct ttattttacc cttggcttag gggtgcaata     180
   aaacttatta taactggaca atatgaaaag                                      210

<210>  17
<211>  1029
<212>  DNA
<213>  Homo sapiens

<400>  17
atgaccagca atttttccca acctgttgtg cagctttgct atgaggatgt gaatggatct      60

tgtattgaaa ctccctattc tcctgggtcc cgggtaattc tgtacacggc gtttagcttt     120

gggtctttgc tggctgtatt tggaaatctc ttagtaatga cttctgttct tcattttaag     180

cagctgcact ctccaaccaa ttttctcatt gcctctctgg cctgtgctga cttcttggta     240

ggtgtgactg tgatgctttt cagcatggtc aggacggtgg agagctgctg gtattttgga     300

gccaaatttt gtactcttca cagttgctgt gatgtggcat ttgttactct ttctgtcctc     360

cacttgtgct tcatctgcat cgacaggtac attgtggtta ctgatcccct ggtctatgct 420
accaagttca ccgtgtctgt gtcgggaatt tgcatcagcg tgtcctggat tctgcctctc 480
acgtacagcg gtgctgtgtt ctacacaggt gtcaatgatg atgggctgga ggaattagta 540
agtgctctca actgcgtagg tggctgtcaa attattgtaa gtcaaggctg ggtgttgata 600
gattttctgt tattcttcat acctaccctt gttatgataa ttctttacag taagattttt 660
cttatagcta aacaacaagc tataaaaatt gaaactacta gtagcaaagt agaatcatcc 720
tcagagagtt ataaaatcag agtggccaag agagagagga aagcagctaa aaccctgggg 780
gtcacggtac tagcatttgt tatttcatgg ttaccgtata cagttgatat attaattgat 840
gcctttatgg gcttcctgac ccctgcctat atctatgaaa tttgctgttg gagtgcttat 900
tataactcag ccatgaatcc tttgatttat gctctatttt atccttggtt taggaaagcc 960
ataaaactta ttttaagtgg agatgtttta aaggctagtt catcaaccat tagtttattt 1020
ttagaataa 1029

<210> 18
<211> 342
<212> PRT
<213> Homo sapiens

<400> 18

Met Thr Ser Asn Phe Ser Gln Pro Val Val Gln Leu Cys Tyr Glu Asp
1               5                   10                  15

Val Asn Gly Ser Cys Ile Glu Thr Pro Tyr Ser Pro Gly Ser Arg Val
            20                  25                  30

Ile Leu Tyr Thr Ala Phe Ser Phe Gly Ser Leu Leu Ala Val Phe Gly
        35                  40                  45

Asn Leu Leu Val Met Thr Ser Val Leu His Phe Lys Gln Leu His Ser
    50                  55                  60

Pro Thr Asn Phe Leu Ile Ala Ser Leu Ala Cys Ala Asp Phe Leu Val
65                  70                  75                  80

Gly Val Thr Val Met Leu Phe Ser Met Val Arg Thr Val Glu Ser Cys
                85                  90                  95

Trp Tyr Phe Gly Ala Lys Phe Cys Thr Leu His Ser Cys Cys Asp Val
            100                 105                 110

Ala Phe Cys Tyr Ser Ser Val Leu His Leu Cys Phe Ile Cys Ile Asp
        115                 120                 125

Arg Tyr Ile Val Val Thr Asp Pro Leu Val Tyr Ala Thr Lys Phe Thr
    130                 135                 140

Val Ser Val Ser Gly Ile Cys Ile Ser Val Ser Trp Ile Leu Pro Leu
145                 150                 155                 160

Thr Tyr Ser Gly Ala Val Phe Tyr Thr Gly Val Asn Asp Asp Gly Leu
                165                 170                 175

Glu Glu Leu Val Ser Ala Leu Asn Cys Val Gly Gly Cys Gln Ile Ile
            180                 185                 190

Val Ser Gln Gly Trp Val Leu Ile Asp Phe Leu Leu Phe Phe Ile Pro
        195                 200                 205

Thr Leu Val Met Ile Ile Leu Tyr Ser Lys Ile Phe Leu Ile Ala Lys
    210                 215                 220

Gln Gln Ala Ile Lys Ile Glu Thr Thr Ser Ser Lys Val Glu Ser Ser
225                 230                 235                 240

Ser Glu Ser Tyr Lys Ile Arg Val Ala Lys Arg Glu Arg Lys Ala Ala
                245                 250                 255

Lys Thr Leu Gly Val Thr Val Leu Ala Phe Val Ile Ser Trp Leu Pro
            260                 265                 270

Tyr Thr Val Asp Ile Leu Ile Asp Ala Phe Met Gly Phe Leu Thr Pro
        275                 280                 285

Ala Tyr Ile Tyr Glu Ile Cys Cys Trp Ser Ala Tyr Tyr Asn Ser Ala
    290                 295                 300

Met Asn Pro Leu Ile Tyr Ala Leu Phe Tyr Pro Trp Phe Arg Lys Ala
305                 310                 315                 320

Ile Lys Leu Ile Leu Ser Gly Asp Val Leu Lys Ala Ser Ser Ser Thr
                325                 330                 335

Ile Ser Leu Phe Leu Glu
            340

<210>  19
<211>  1047
<212>  DNA
<213>  Homo sapiens

<400>  19
atggtgaata atttctccca agctgaggct gtggagctgt gttacaagaa cgtgaacgaa      60

tcctgcatta aaactcctta ctcgccaggt cctcgatcta tcctctacgc cgtccttggt     120

tttggggctg tgctggcagc gtttggaaac ttactggtca tgattgctat ccttcacttc     180

aaacaactgc acacacctac aaactttctg attgcgtcgc tggcctgtgc tgacttcttg 240

gtgggagtca ctgtgatgcc cttcagcaca gtgaggtctg tggagagctg ttggtacttt 300

ggggacagtt actgtaaatt ccatacatgt tttgacacat ccttctgttt tgcttcttta 360

tttcatttat gctgtatctc tgttgataga tacattgctg ttactgatcc tctgacctat 420

ccaaccaagt ttactgtgtc agtttcaggg atatgcattg ttctttcctg gttcttttct 480

gtcacataca gcttttcgat cttttacacg ggagccaacg aagaaggaat tgaggaatta 540

gtagttgctc taacctgtgt aggaggctgc caggctccac tgaatcaaaa ctgggtccta 600

ctttgttttc ttctattctt tatacccaat gtcgccatgg tgtttatata cagtaagata 660

tttttggtgg ccaagcatca ggctaggaag atagaaagta cagccagcca agctcagtcc 720

tcctcagaga gttacaagga aagagtagca aaaagagaga gaaaggctgc caaaaccttg 780

ggaattgcta tggcagcatt tcttgtctct tggctaccat acctcgttga tgcagtgatt 840

gatgcttata tgaattttat aactcctcct tatgtttatg agattttagt ttggtgtgtt 900

tattataatt cagctatgaa ccccttgatt tatgctttct tttaccaatg gtttgggaag 960

gcaataaaac ttattgtaag cggcaaggtc ttaaggactg attcgtcaac aactaattta 1020

ttttctgaag aagtagagac agattaa 1047

<210> 20
<211> 348
<212> PRT
<213> Homo sapiens

<400> 20

Met Val Asn Asn Phe Ser Gln Ala Glu Ala Val Glu Leu Cys Tyr Lys
1               5                   10                  15

Asn Val Asn Glu Ser Cys Ile Lys Thr Pro Tyr Ser Pro Gly Pro Arg
            20                  25                  30

Ser Ile Leu Tyr Ala Val Leu Gly Phe Gly Ala Val Leu Ala Ala Phe
        35                  40                  45

Gly Asn Leu Leu Val Met Ile Ala Ile Leu His Phe Lys Gln Leu His
    50                  55                  60

Thr Pro Thr Asn Phe Leu Ile Ala Ser Leu Ala Cys Ala Asp Phe Leu
65                  70                  75                  80

Val Gly Val Thr Val Met Pro Phe Ser Thr Val Arg Ser Val Glu Ser
                85                  90                  95

Cys Trp Tyr Phe Gly Asp Ser Tyr Cys Lys Phe His Thr Cys Phe Asp
            100                 105                 110

Thr Ser Phe Cys Phe Ala Ser Leu Phe His Leu Cys Cys Ile Ser Val
            115                 120                 125

Asp Arg Tyr Ile Ala Val Thr Asp Pro Leu Thr Tyr Pro Thr Lys Phe
    130                 135                 140

Thr Val Ser Val Ser Gly Ile Cys Ile Val Leu Ser Trp Phe Phe Ser
145                 150                 155                 160

Val Thr Tyr Ser Phe Ser Ile Phe Tyr Thr Gly Ala Asn Glu Glu Gly
                165                 170                 175

Ile Glu Glu Leu Val Val Ala Leu Thr Cys Val Gly Gly Cys Gln Ala
            180                 185                 190

Pro Leu Asn Gln Asn Trp Val Leu Leu Cys Phe Leu Leu Phe Phe Ile
        195                 200                 205

Pro Asn Val Ala Met Val Phe Ile Tyr Ser Lys Ile Phe Leu Val Ala
    210                 215                 220

Lys His Gln Ala Arg Lys Ile Glu Ser Thr Ala Ser Gln Ala Gln Ser
225                 230                 235                 240

Ser Ser Glu Ser Tyr Lys Glu Arg Val Ala Lys Arg Glu Arg Lys Ala
                245                 250                 255

Ala Lys Thr Leu Gly Ile Ala Met Ala Ala Phe Leu Val Ser Trp Leu
            260                 265                 270

Pro Tyr Leu Val Asp Ala Val Ile Asp Ala Tyr Met Asn Phe Ile Thr
        275                 280                 285

Pro Pro Tyr Val Tyr Glu Ile Leu Val Trp Cys Val Tyr Tyr Asn Ser
    290                 295                 300

Ala Met Asn Pro Leu Ile Tyr Ala Phe Phe Tyr Gln Trp Phe Gly Lys
305                 310                 315                 320

Ala Ile Lys Leu Ile Val Ser Gly Lys Val Leu Arg Thr Asp Ser Ser
                325                 330                 335

Thr Thr Asn Leu Phe Ser Glu Glu Val Glu Thr Asp
            340                 345

<210>  21
<211>  18
<212>  DNA
<213>  Homo sapiens

<400>  21

atgatgccct tttgccac                                                    18

<210>  22
<211>  29
<212>  DNA
<213>  Homo sapiens

<400>  22
ttatgaactc aattccaaaa ataatttac                                        29

<210>  23
<211>  22
<212>  DNA
<213>  Homo sapiens

<400>  23
catctacatc tggggtatct tg                                               22

<210>  24
<211>  21
<212>  DNA
<213>  Homo sapiens

<400>  24
tgcttcaatt tattcatgca g                                                21

<210>  25
<211>  24
<212>  DNA
<213>  Homo sapiens

<400>  25
atggatctaa cttatattcc cgaa                                             24

<210>  26
<211>  22
<212>  DNA
<213>  Homo sapiens

<400>  26
ttaatgtgct tcaggaaaca aa                                               22

<210>  27
<211>  19
<212>  DNA
<213>  Homo sapiens

<400>  27
atgatgaatt tgcctgacc                                                   19

<210>  28
<211>  19
<212>  DNA
<213>  Homo sapiens

<400>  28
ctaagcatgg gcagaaaac                                                   19

<210>  29
<211>  20
<212>  DNA
<213>  Homo sapiens

<400>  29
atgagagctg tcttcatcca                                                    20


<210>  30
<211>  23
<212>  DNA
<213>  Homo sapiens

<400>  30
tcattcttgg tacaaatcaa cag                                                23


<210>  31
<211>  19
<212>  DNA
<213>  Homo sapiens

<400>  31
atgagcagca attcatccc                                                     19


<210>  32
<211>  28
<212>  DNA
<213>  Homo sapiens

<400>  32
ttatatatgt tcagaaaaca aattcatg                                           28


<210>  33
<211>  19
<212>  DNA
<213>  Homo sapiens

<400>  33
atgaccagca atttttccc                                                     19


<210>  34
<211>  29
<212>  DNA
<213>  Homo sapiens

<400>  34
ttattctaaa aataaactaa tggttgatg                                          29


<210>  35
<211>  22
<212>  DNA
<213>  Homo sapiens

<400>  35
atggtgaata atttctccca ag                                                 22


<210>  36
<211>  28
<212>  DNA

<213>  Homo sapiens

<400>  36
ttaatctgtc tctacttctt cagaaaat                                          28